(19) Europäisches Patentamt European Patent Office Office européen des brevets

(11) **EP 4 477 670 A1**

(12) **EUROPEAN PATENT APPLICATION**
published in accordance with Art. 153(4) EPC

(43) Date of publication:
18.12.2024 Bulletin 2024/51

(21) Application number: 23752393.1

(22) Date of filing: 09.02.2023

(51) International Patent Classification (IPC):
*C07K 16/46* (2006.01)   *C12N 15/13* (2006.01)
*C12N 15/63* (2006.01)   *A61K 39/395* (2006.01)
*A61P 35/00* (2006.01)

(52) Cooperative Patent Classification (CPC):
A61K 39/395; A61P 35/00; C07K 16/00;
C07K 16/46; C12N 15/63

(86) International application number:
PCT/CN2023/075148

(87) International publication number:
WO 2023/151613 (17.08.2023 Gazette 2023/33)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 10.02.2022 CN 202210123816

(71) Applicant: Shanghai Qilu Pharmaceutical
Research and
Development Centre Ltd.
Shanghai 201203 (CN)

(72) Inventors:
• YANG, Liuqing
Shanghai 201203 (CN)

• LI, Ruimei
Shanghai 201203 (CN)
• QIAN, Hongliang
Shanghai 201203 (CN)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

Remarks:
The complete document including Reference
Table(s) and the Sequence Listing(s) can be
downloaded from the EPO website

(54) **BISPECIFIC ANTIGEN-BINDING MOLECULE AND USE THEREOF**

(57) The present disclosure provides a form of bispecific antigen-binding molecule, and also provides a bispecific antigen-binding molecule constructed against DLL3 on the basis of the form, a pharmaceutical composition comprising the bispecific antigen-binding molecule and related use thereof in the treatment of tumor.

Bispecific antibody 1

Anti-DLL3 nanobody
Light chain variable region of anti-CD3 antibody
Heavy chain variable region of anti-CD3 antibody
Fc domain

FIG. 1A

Bispecific antibody 2

Fab fragment of anti-DLL3 full-length antibody
Light chain variable region of anti-CD3 antibody
Heavy chain variable region of anti-CD3 antibody
Fc domain with "knob" and "hole" structures

FIG. 1B

FIG. 1

EP 4 477 670 A1

Processed by Luminess, 75001 PARIS (FR)

**Description**

The present disclosure claims the priority of Chinese patent application No. 2022101238168, with an application date of 2022/02/10, and the above-mentioned Chinese patent application is incorporated into the present disclosure by reference in its entirety.

## TECHNICAL FIELD

The present disclosure belongs to the field of immunology and relates to a bispecific antigen-binding molecule. The present disclosure also relates to the related encoding nucleic acid, vector, host cell, drug, and related use in the treatment of cancer.

## BACKGROUND

Monoclonal antibodies have been widely used to treat a variety of diseases including tumors, but the long-term effectiveness of monoclonal antibody therapy has been limited by mechanisms such as tumor immune evasion. One solution is to develop bispecific antibodies that can bind to two different antigens or epitopes, thereby blocking different signaling pathways in tumorigenesis at the same time, or can directly target immune cells to tumor cells, thus potentially producing greater cytotoxicity and better avoiding immune evasion. Various forms of bispecific antibodies have been developed, however there is still a need to develop new forms of bispecific antibodies.

Small cell lung cancer (SCLC) accounts for approximately 15%-20% of lung cancers and is characterized by high vascular distribution, rapid tumor growth, genomic instability, and early metastatic transmission. It is estimated that more than 234,000 cases of small cell lung cancer are diagnosed each year, causing approximately 250,000 deaths worldwide each year. It is almost impossible to cure SCLC completely by local treatment such as surgery, radiotherapy or a combination of both. Platinum-based combination chemotherapy remains the cornerstone of the treatment. Although response rates to first-line chemotherapy are high, recurrence rates are also high, with median survival of only 14-20 months for patients in localized stage and 9-11 months for patients in extensive stage, and shorter survival and few treatment options for patients in relapse. Topotecan, the only FDA-recommended drug, is limited by its hematological toxicity, and the treatment response rate is unsatisfactory, only 5-24%, with a median survival of less than 25 weeks. There is currently no specific third-line treatment recommended, so a new and effective therapeutic drug is urgently needed.

Delta-like protein 3 (Delta-like 3), also known as DLL3, is a protein encoded by the DLL3 gene and is a ligand of the Notch family. The protein is involved in affecting the Notch regulatory signaling pathway, causing signals from the Notch pathway to prompt unrestricted growth of tumor cells. DLL3 was found to be expressed on the surface of tumor cells in about 85% of patients with small cell lung cancer and large cell neuroendocrine carcinoma, and was also highly expressed in glioblastoma multiforme, melanoma, pancreatic cancer and rectal cancer. However, DLL3 is not expressed in healthy tissues and non-neuroendocrine tumors, so DLL3 is an ideal target for small cell lung cancer.

Bispecific antibodies targeting CD3 antigen and tumor associated antigen can bring T cells and tumor cells closer in space, and use T cells to achieve specific killing effect on tumor cells. Such antibodies are called T cell-engagers (TCE bispecific antibodies). Among the bispecific antibodies in clinical stage, nearly half of the candidates target the CD3 antigen, studying the safety and efficacy of TCE bispecific antibodies. Although relevant research has made some progress, there are still many challenges, such as how to balance anti-tumor activity and safety. It is particularly important to develop new T cell bispecific antibodies with high efficiency and low toxicity.

Therefore, it is necessary to develop new forms of bispecific antibodies and use them to further develop bispecific antibodies with high efficiency and low toxicity.

## SUMMARY

The first object of the present disclosure is to provide a bispecific antigen-binding molecule with a novel form, in which the second antigen-binding portion is wrapped between the first antigen-binding portion and the Fc structure, which can reduce the exposure of the second antigen-binding portion and thus reduce the risk of non-specific cytokine release. One form of the bispecific antigen-binding molecule comprises:

(A) a first polypeptide comprising: (i) an antigen-binding fragment (Fab) heavy chain domain specifically directing to a first antigen, (ii) a single-chain antibody (scFv) domain capable of specifically binding to a second antigen, and (iii) a first Fc domain;

(B) a second polypeptide comprising: a second Fc domain;

(C) a third polypeptide comprising: an antigen-binding fragment (Fab) light chain domain specifically directing to the first antigen;

the antigen-binding fragment (Fab) heavy chain domain and the antigen-binding fragment (Fab) light chain domain form a first binding site against the first antigen, the single-chain antibody (scFv) domain forms a second binding site against the second antigen, and the first Fc domain and the second Fc domain being associated with each other.

In one embodiment, the single-chain antibody (scFv) domain comprises a second heavy chain variable region and a second light chain variable region;

preferably, the second heavy chain variable region is connected to the second light chain variable region via a first linker or directly, wherein:

the C-terminal of the second heavy chain variable region is fused with the N-terminal of the first linker, and the C-terminal of the first linker is fused with the N-terminal of the second light chain variable region; or

the C-terminal of the second light chain variable region is fused with the N-terminal of the first linker and the C-terminal of the first linker is fused with the N-terminal of the second heavy chain variable region; or

the C-terminal of the second heavy chain variable region is fused with the N-terminal of the second light chain variable region; or

the C-terminal of the second light chain variable region is fused with the N-terminal of the second heavy chain variable region;

more preferably, the first linker comprises an amino acid sequence $(G_4S)_n$, wherein n is any integer from 1 to 10.

In one embodiment, the first Fc domain comprises a first CH2 domain and a first CH3 domain of an immunoglobulin, the C-terminal of the first CH2 domain being fused with the N-terminal of the first CH3 domain; the second Fc domain comprises a second CH2 domain and a second CH3 domain of an immunoglobulin, the C-terminal of the second CH2 domain being fused with the N-terminal of the second CH3 domain;

preferably, the first CH3 domain comprises a "knob" structure and the second CH3 domain comprises a "hole" structure; more preferably, the "knob" structure comprises amino acid substitutions S354C and T366W, the "hole" structure comprises amino acid substitutions Y349C, T366S, L368A and Y407V;

preferably, the Fc domain is derived from IgG1;

preferably, the single-chain antibody (scFv) domain is connected to the first Fc domain via a second linker or directly, wherein:

the C-terminal of the single-chain antibody (scFv) domain is fused with the N-terminal of the second linker, and the C-terminal of the second linker is fused with the N-terminal of the first Fc domain; or

the C-terminal of the single-chain antibody (scFv) domain is fused with the N-terminal of the first Fc domain;

more preferably, the second linker comprises an amino acid sequence EPKSS (SEQ ID NO: 34).

In one embodiment, the heavy chain domain of the antigen-binding fragment (Fab) comprises a first heavy chain variable region and a CH1 domain of an immunoglobulin, and the C-terminal of the first heavy chain variable region is fused with the N-terminal of the CH1 domain; the light chain domain of the antigen-binding fragment (Fab) comprises a first light chain variable region and a light chain constant region of an immunoglobulin, and the C-terminal of the first light chain variable region is fused with the N-terminal of the light chain constant region;

preferably, the antigen-binding fragment (Fab) heavy chain domain is connected to the single-chain antibody (scFv) domain by a third linker or directly, wherein:

the C-terminal of the heavy chain domain of the antigen-binding fragment (Fab) is fused with the N-terminal of the third linker, and the C-terminal of the third linker is fused with the N-terminal of the single-chain antibody (scFv) domain; or

the C-terminal of the single-chain antibody (scFv) domain is fused with the N-terminal of the third linker, and the C-terminal of the third linker is fused with the N-terminal of the antigen-binding fragment (Fab) heavy chain domain; or

the C-terminal of the heavy chain domain of an antigen-binding fragment (Fab) is fused with the N-terminal of the single-chain antibody (scFv) domain; or

the C-terminal of the single-chain antibody (scFv) domain is fused with the N-terminal of the antigen-binding fragment (Fab) heavy chain domain;

more preferably, the third linker comprises an amino acid sequence $(G_4S)_n$, wherein n is any integer from 1 to 10.

In one embodiment, the first polypeptide comprises the following structure: Fab heavy chain domain-the third linker-scFv domain-the second linker-the first Fc domain or scFv domain-the third linker-Fab heavy chain domain-the second linker-the first Fc domain;
preferably, the first polypeptide comprises the following structure:

the first heavy chain variable region-CH1-the third linker-the second heavy chain variable region-the first linker-the second light chain variable region-the second linker-the first CH2-the first CH3; or

the first heavy chain variable region-CH1-the third linker-the second light chain variable region-the first linker-the second heavy chain variable region-the second linker-the first CH2-the first CH3; or

the second heavy chain variable region-the first linker-the second light chain variable region-CH1-the third linker-the first heavy chain variable region-the second linker-the first CH2-the first CH3; or

the second light chain variable region-the first linker-the second heavy chain variable region-CH1-the third linker-the first heavy chain variable region-the second linker-the first CH2-the first CH3;

the second polypeptide comprises the following structure: the second CH2- the second CH3; the third polypeptide comprises the following structure: the first light chain variable region-light chain constant region.

In one embodiment, the bispecific antigen-binding molecule comprises one or more amino acid substitutions selected from the group consisting of: (i) L234A and L235A; (ii) H435R; preferably, the H435R substitution is a substitution on the second polypeptide.

In one embodiment, the second polypeptide is CD3, preferably CD3ε; preferably, the single-chain antibody (scFv) domain comprises HCDR1 with the sequence as shown in SEQ ID NO: 24, HCDR2 with the sequence as shown in SEQ ID NO: 25, HCDR3 with the sequence as shown in SEQ ID NO: 26, LCDR1 with the sequence as shown in SEQ ID NO: 27, LCDR2 with the sequence as shown in SEQ ID NO: 28, and LCDR3 with the sequence as shown in SEQ ID NO: 29;

more preferably, the single-chain antibody (scFv) domain comprises a second heavy chain variable region with the sequence as shown in SEQ ID NO: 21 and a second light chain variable region with the sequence as shown in SEQ ID NO: 22;

more preferably, the single-chain antibody (scFv) domain comprises the amino acid sequence as shown in SEQ ID NO: 23.

In one embodiment, the first Fc domain comprises the amino acid sequence as shown in SEQ ID NO: 31; the second Fc domain comprises the amino acid sequence as shown in SEQ ID NO: 3.

In one embodiment, the first antigen is DLL3; preferably, the antigen-binding fragment (Fab) heavy chain domain comprises HCDR1 with the sequence as shown in SEQ ID NO: 13, HCDR2 with the sequence as shown in SEQ ID NO: 14 and HCDR3 with the sequence as shown in SEQ ID NO: 15; the antigen-binding fragment (Fab) light chain domain comprises LCDR1 with the sequence as shown in SEQ ID NO: 16, LCDR2 with the sequence as shown in SEQ ID NO: 17 and LCDR3 with the sequence as shown in SEQ ID NO: 18;

more preferably, the antigen-binding fragment (Fab) heavy chain domain comprises a first heavy chain variable region with the sequence as shown in SEQ ID NO: 11; the antigen-binding fragment (Fab) light chain domain comprises a first light chain variable region with the sequence as shown in SEQ ID NO: 12;

more preferably, the antigen-binding fragment (Fab) heavy chain domain comprises the amino acid sequence as shown in SEQ ID NO: 33; the antigen-binding fragment (Fab) light chain domain comprises the amino acid sequence as shown in SEQ ID NO: 4.

In one embodiment, the first polypeptide comprises the amino acid sequence as shown in SEQ ID NO: 2; the second polypeptide comprises the amino acid sequence as shown in SEQ ID NO: 3; the third polypeptide comprises the amino acid sequence as shown in SEQ ID NO: 4.

Another form of the bispecific antigen-binding molecule is comprising two homologous polypeptides, each of the polypeptides comprising: (i) a nanobody (VHH) domain capable of specifically binding to a first antigen, (ii) a single-chain antibody (scFv) domain capable of specifically binding to a second antigen, and (iii) an Fc domain; the Fc domains of the two polypeptides being associated with each other.

In one embodiment, the single-chain antibody (scFv) domain comprises a heavy chain variable region and a light chain variable region;

preferably, the heavy chain variable region is connected to the light chain variable region via a first linker or directly, wherein:

the C-terminal of the heavy chain variable region is fused with the N-terminal of the first linker, and

the C-terminal of the first linker is fused with the N-terminal of the light chain variable region; or

the C-terminal of the light chain variable region is fused with the N-terminal of the first linker, and the C-terminal of the first linker is fused with the N-terminal of the heavy chain variable region; or

the C-terminal of the heavy chain variable region is fused with the N-terminal of the light chain variable region; or

the C-terminal of the light chain variable region is fused with the N-terminal of the heavy chain variable region;

more preferably, the first linker comprises an amino acid sequence $(G_4S)_n$, wherein n is any integer from 1 to 10.

In one embodiment, the Fc domain comprises a CH2 domain and a CH3 domain of an immunoglobulin, the C-terminal of the CH2 domain being fused with the N-terminal of the CH3 domain;

preferably, the Fc domain is derived from IgG1;

preferably, the Fc domain is connected to the single-chain antibody (scFv) domain via a second linker or directly, wherein:

the C-terminal of the single-chain antibody (scFv) domain is fused with the N-terminal of the second linker, and the C-terminal of the second linker is fused with the N-terminal of the Fc domain; or

the C-terminal of the single-chain antibody (scFv) domain is fused with the N-terminal of the Fc domain;

more preferably, the second linker comprises an amino acid sequence EPKSS (SEQ ID NO: 34).

In one embodiment, the nanobody (VHH) domain is connected to the single-chain antibody (scFv) domain by a third linker or directly, wherein:

the C-terminal of the nanobody (VHH) domain is fused with the N-terminal of the third linker, and

the C-terminal of the third linker is fused with the N-terminal of the single-chain antibody (scFv) domain; or

the C-terminal of the single-chain antibody (scFv) domain is fused with the N-terminal of the third linker, and the C-terminal of the third linker is fused with the N-terminal of nanobody (VHH) domain; or

the C-terminal of the nanobody (VHH) domain is fused with the N-terminal of the single-chain antibody (scFv) domain; or

the C-terminal of the single-chain antibody (scFv) domain is fused with the N-terminal of nanobody (VHH) domain;

more preferably, the third linker comprises an amino acid sequence $(G_4S)_n$, wherein n is any integer from 1 to 10.

In one embodiment, each of the polypeptides comprises the following structure:

VHH domain-the third linker-scFv domain-the second linker-Fc domain or scFv domain-the third linker-VHH domain-the second linker-Fc domain;

preferably, each of the polypeptides comprises the following structure:

VHH domain-the third linker-heavy chain variable region-the first linker-light chain variable region-the second linker-CH2-CH3, or

VHH domain-the third linker-light chain variable region-the first linker-heavy chain variable region-the second linker-CH2-CH3, or

heavy chain variable region-the first linker-light chain variable region-the third linker-VHH domain-the second linker-CH2-CH3, or

light chain variable region-the first linker-heavy chain variable region-the third linker-VHH domain-the second linker-CH2-CH3.

In one embodiment, the bispecific antigen-binding molecule comprises the following amino acid substitutions: L234A and L235A.

In one embodiment, the second antigen is CD3, preferably CD3ε; preferably, the single-chain antibody (scFv) domain comprises HCDR1 with the sequence as shown in SEQ ID NO: 24, HCDR2 with the sequence as shown in SEQ ID NO: 25, HCDR3 with the sequence as shown in SEQ ID NO: 26, LCDR1 with the sequence as shown in SEQ ID NO: 27, LCDR2 with the sequence as shown in SEQ ID NO: 28, and LCDR3 with the sequence as shown in SEQ ID NO: 29;

more preferably, the single-chain antibody (scFv) domain comprises a heavy chain variable region with the sequence as shown in SEQ ID NO: 21 and a light chain variable region with the sequence as shown in SEQ ID NO: 22;

more preferably, the single-chain antibody (scFv) domain comprises the amino acid sequence as shown in SEQ ID NO: 23.

In one embodiment, the Fc domain comprises the amino acid sequence as shown in SEQ ID NO: 30.

In one embodiment, the first antigen is DLL3; preferably, the nanobody (VHH) domain comprises HCDR1 with the sequence as shown in SEQ ID NO: 6, HCDR2 with the sequence as shown in SEQ ID NO: 7 and HCDR3 with the sequence as shown in SEQ ID NO: 8;

more preferably, the nanobody (VHH) domain comprises the amino acid sequence as shown in SEQ ID NO: 32.

In one embodiment, each polypeptide of the bispecific antigen-binding molecule comprises the amino acid sequence as shown in SEQ ID NO: 1.

A second object of the present disclosure is to provide an anti-DLL3 (preferably anti-DLL3 and CD3) bispecific antigen-binding molecule, which capable of specifically killing tumor cells expressing high level of DLL3 by recruiting T cells to tumor sites by targeting immune cell surface antigens (preferably CD3 antigen) with low level of cytokine release in vitro and good safety.

Based on this, the present disclosure provides a bispecific antigen-binding molecule that binds to a specific epitope, and the epitope to which the bispecific antigen-binding molecule is capable of binding to is:

(i) identical to or overlaps with an epitope to which a nanobody comprising HCDR1 with the sequence as shown in SEQ ID NO: 6, HCDR2 with the sequence as shown in SEQ ID NO: 7 and HCDR3 with the sequence as shown in SEQ ID NO: 8 directs; or

(ii) identical to or overlaps with an epitope to which an antibody comprising HCDR1 with the sequence as shown in SEQ ID NO: 13, HCDR2 with the sequence as shown in SEQ ID NO: 14, HCDR3 with the sequence as shown in SEQ ID NO: 15, LCDR1 with the sequence as shown in SEQ ID NO: 16, LCDR2 with the sequence as shown in SEQ ID NO: 17, and LCDR3 with the sequence as shown in SEQ ID NO: 18 directs.

In one embodiment, the epitope to which the bispecific antigen-binding molecule is capable of binding to is:

(i) identical to or overlaps with an epitope to which a nanobody comprising the amino acid sequence as shown in SEQ ID NO: 32 directs; or

(ii) identical to or overlaps with an epitope to which an antibody comprising a first heavy chain variable region with the sequence as shown in SEQ ID NO: 11 and a first light chain variable region with the sequence as shown in SEQ ID NO: 12 directs.

In one embodiment, the bispecific antigen-binding molecule is capable of binding to CD3, preferably capable of binding to CD3ε.

The present disclosure also provides a bispecific antigen-binding molecule comprising:

(A) a first binding portion capable of specifically binding to DLL3; and

(B) a second binding portion, the antigen or epitope to which the second binding portion specifically binds is different from that of the first binding portion;

the first binding portion comprises:

(i) HCDR1 with the sequence as shown in SEQ ID NO: 6, HCDR2 with the sequence as shown in SEQ ID NO: 7 and HCDR3 with the sequence as shown in SEQ ID NO: 8; or

(ii) HCDR1 with the sequence as shown in SEQ ID NO: 13, HCDR2 with the sequence as shown in SEQ ID NO: 14, HCDR3 with the sequence as shown in SEQ ID NO: 15, LCDR1 with the sequence as shown in SEQ ID NO: 16, LCDR2 with the sequence as shown in SEQ ID NO: 17 and LCDR3 with the sequence as shown in SEQ ID NO: 18.

In one embodiment, the first binding portion comprises:

(i) the amino acid sequence as shown in SEQ ID NO: 32; or

(ii) a first heavy chain variable region with the sequence as shown in SEQ ID NO: 11 and a first light chain variable region with the sequence as shown in SEQ ID NO: 12.

In one embodiment, the second binding portion is capable of binding to CD3, preferably to CD3ε.

The present disclosure also provides a nucleic acid molecule encoding the aforementioned bispecific antigen-binding molecule.

The disclosure also provides a expression vector comprising the foregoing nucleic acid molecule.

The present disclosure also provides a host cell comprising the aforementioned nucleic acid molecule or expression vector; preferably, the host cell is a prokaryotic cell or a eukaryotic cell; the prokaryotic cell is preferably Escherichia coli; the eukaryotic cell is preferably a mammalian cell or yeast; more preferably, the mammalian cell is a CHO cell, Expi293 or HEK293 cell.

The present disclosure also provides a method for preparing a bispecific antigen-binding molecule, the method comprises culturing the aforementioned host cell under a suitable condition.

The present disclosure also provides an antibody-drug conjugate forming by conjugating the aforementioned bispecific antigen-binding molecule with other biologically active molecule; preferably, the other biologically active molecule is a small molecule drug; preferably, the bispecific antigen-binding molecule and the other biologically active molecule are connected via a linker.

The present disclosure also provides a pharmaceutical composition comprising the aforementioned bispecific antigen-binding molecule, nucleic acid molecule, expression vector, host cell, and/or antibody-drug conjugate.

In one embodiment, the pharmaceutical composition further comprises a pharmaceutically acceptable carrier.

In one embodiment, the pharmaceutical composition further comprises one or more additional therapeutic agents.

The present disclosure also provides the use of the aforementioned bispecific antigen-binding molecule, nucleic acid molecule, expression vector, host cell and/or antibody-drug conjugate in the manufacture of a medicament for treating, alleviating and/or preventing a tumor. Preferably, the tumor is a DLL3-positive tumor.

The present disclosure also provides a method for inducing death of a cell expressing DLL3, the method comprises contacting the cell with the aforementioned bispecific antigen-binding molecule, nucleic acid molecule, expression vector, host cell, antibody-drug conjugate and/or pharmaceutical composition, wherein the cell expressing DLL3 is a tumor cell.

The present disclosure also provides a method for treating a disease associated with expression of DLL3 in a subject, the method comprises administering the aforementioned bispecific antigen-binding molecule, nucleic acid molecule, expression vector, host cell, antibody-drug conjugate, and/or pharmaceutical composition to a subject in need thereof. Preferably, the disease is a tumor.

Preferably, the tumor/tumor cell of the present disclosure is selected from: small cell lung cancer, glioblastoma, neuroendocrine cancer, melanoma, pancreatic cancer, rectal cancer and a metastatic cancer of the above-mentioned tumor.

In one embodiment, the method further comprises administering an additional therapeutic agent to the subject.

The substitution referred to in the present disclosure are preferably indicated using the EU numbering system.

The technical solution disclosed in the present disclosure has the following beneficial effects: while maintaining a strong ability of killing tumor cells in vitro, the release level of non-specific cytokines in vitro is weak or non-existent, and the safety is good.

## DESCRIPTION OF THE DRAWINGS

The drawings further illustrate the novel features disclosed in the present description. The features and advantages disclosed in the present description will be better understood with reference to these drawings, but it should be understood that these drawings are only used to illustrate particular embodiments of the principles disclosed herein and are not intended to limit the scope of the appended claims.

FIG. 1 shows the structures of two bispecific antibodies constructed in the Examples. FIG. 1A shows Bispecific antibody 1 and FIG. 1B shows Bispecific antibody 2.

FIG. 2 shows the binding of Bispecific antibody 1 and Bispecific antibody 2 to SHP-77 cells expressing hDLL3.

FIG. 3 shows the binding of Bispecific antibody 1 and Bispecific antibody 2 to Jurkat cells that naturally express hCD3.

FIG. 4 shows the results of cytotoxicity assay (TDCC) of $CD3^+$ T cells mediated by Bispecific antibody 1 and Bispecific antibody 2 against SHP-77 cells.

FIG. 5 shows the results of cytotoxicity assay (TDCC) of $CD3^+$ T cells mediated by Bispecific antibody 1 and Bispecific antibody 2 against NCI-H82 cells.

FIG. 6 shows the experimental results of cytokine release of Bispecific antibody 1 and Bispecific antibody 2 in the presence and absence of target cells. FIGs. 6A-6E show cytokine release results of Bispecific antibody 1 molecule, respectively IFNγ, TNFα, IL-10, IL-6 and IL4; FIGs. 6F-6J show cytokine release results of Bispecific antibody 2 molecule, respectively IFNγ, TNFα, IL-10, IL-6 and IL4.

FIG. 7 is a schematic diagram of tumor inoculation position in mice in the in vivo pharmacodynamic experiment of Bispecific antibody 2.

FIG. 8 shows the effect of Bispecific antibody 2 on tumor growth weight in the SHP-77 small cell lung cancer model.

## Detailed Description of the Invention

## Definitions

All publications, patents, and patent applications mentioned in the present description are incorporated herein by reference to the same extent as if each individual publication, patent, or patent application was specifically and individually indicated to be incorporated herein by reference.

Before the present disclosure is described in detail below, it is to be understood that the present disclosure is not limited to the particular methodologies, protocols, and reagents described herein, as these may vary. It is also to be understood that the term used herein is for the purpose of describing particular embodiments only and is not intended to limit the scope of the present disclosure. Unless defined otherwise, all technical and scientific terms used herein have the same meaning as commonly understood by one of ordinary skill in the art to which the present disclosure belongs.

Certain embodiments disclosed herein include numerical ranges, and certain aspects of the present disclosure may be described in terms of ranges. Unless otherwise indicated, it should be understood that numerical ranges or the method of describing in a range is only for the purposes of brevity and convenience and should not be considered as strict limitations on the scope of the present disclosure. Accordingly, description in a range format should be considered to have specifically disclosed all possible sub-ranges and all possible specific numerical points within that range, as if such sub-ranges and numerical points were expressly written herein. The above principles apply equally regardless of the magnitude of the values stated. When a range description is used, the range includes the endpoints of the range.

The term "about" when referring to a measurable value, such as an amount, a temporal duration, etc., is meant to encompass variations of ± 20%, or in some cases ± 10%, or in some cases ± 5%, or in some cases ± 1%, or in some cases ± 0.1% of the specified value.

As used herein, the term "antibody" may include an intact antibody (e.g., a full-length monoclonal antibody) and any antigen-binding fragment (i.e., antigen-binding portion) thereof or a single chain thereof, and may also include a product having antigen-specific binding ability formed by modification (e.g., connection to other peptide segments, rearrangement of functional units, etc.) on the basis of an intact antibody or an antigen-binding fragment thereof or a single chain thereof.

In one embodiment, an antibody typically refers to a Y-type tetrameric protein comprising two heavy (H) polypeptide chains and two light (L) polypeptide chains held together by covalent disulfide bonds and non-covalent interactions. Natural IgG antibodies have such structure. Each light chain consists of one variable domain (VL) and one constant domain (CL). Each heavy chain contains one variable domain (VH) and one constant region.

Five major classes of antibodies are known in the art: IgA, IgD, IgE, IgG and IgM. The corresponding heavy chain constant domains are referred to as $\alpha$, $\delta$, $\varepsilon$, $\gamma$ and $\mu$, respectively. IgG and IgA can be further divided into different subclasses, for example IgG can be divided into IgG1, IgG2, IgG3, IgG4, and IgA can be divided into IgA1 and IgA2. The light chains of antibodies from any vertebrate species can be assigned to one of two clearly distinct types, called $\kappa$ and $\lambda$, based on the amino acid sequences of their constant domains.

In the case of IgG, IgA and IgD antibodies, the constant region comprises three domains called CH1, CH2 and CH3 (IgM and IgE have a fourth domain CH4). In the IgG, IgA, and IgD classes, the CH1 and CH2 domains are separated by a flexible hinge region, which is a proline- and cysteine-rich knob of variable length. Each class of antibodies further comprises inter-chain and intra-chain disulfide bonds formed by paired cysteine residues.

The term "variable region" or "variable domain" shows significant variation in amino acid composition from one antibody to another and is primarily responsible for antigen recognition and binding. The variable regions of each light/heavy chain pair form the antibody combining site such that an intact IgG antibody has two binding sites (i.e., it is bivalent). The variable region of the heavy chain (VH) and the variable region of the light chain (VL) each comprises three regions of extreme variability, referred to as hypervariable regions (HVRs) or, more commonly, complementarity determining regions (CDRs). VH and VL each has four framework regions (FRs), denoted by FR1, FR2, FR3, FR4, respectively. Thus, CDR and FR sequences typically appear in the following sequences of heavy chain variable domains (or light chain variable domains): FR1-HCDR1 (LCDR1)-FR2-HCDR2 (LCDR2)-FR3-HCDR3 (LCDR3)-FR4.

The term "nanobody" refers to an amino acid sequence having the following (generic) structure: FR1-CDR1-FR2-CDR2-FR3-CDR3-FR4. wherein FR1-FR4 refer to framework regions (Frame) 1-4, respectively, and wherein CDR1-CDR3 refer to complementarity determining regions 1-3, respectively. "VHH" refers to variable antigen-binding domains of heavy chain antibodies from camelids (camel, dromedary, llama, alpaca, etc.).

The term "scFv" refers to a fusion protein comprising at least one antibody fragment comprising a variable region of a light chain and at least one antibody fragment comprising a variable region of a heavy chain, wherein the light chain and heavy chain variable regions are contiguous (e.g., via a synthetic linker such as a short flexible polypeptide linker) and can be expressed as a single chain polypeptide, and wherein the scFv retains the specificity of the intact antibody from which it is derived. Unless specified, the scFv may have the VL and VH variable regions described in any order (e.g., relative to the N-terminal and C-terminal of the polypeptide), and the scFv may include VL-linker-VH or may include VH-linker-VL.

The term "Fc" is used to define the C-terminal region of an immunoglobulin heavy chain, and the C-terminal region comprises at least a portion of a constant region. The term includes native sequence Fc regions and variant Fc regions. Although the boundaries of the Fc region of an IgG heavy chain may vary slightly, the Fc region of a human IgG heavy chain is generally defined as extending from Cys226 or Pro230 to the carboxy terminal of the heavy chain, e.g., the IgG Fc domain comprises IgG CH2 and IgG CH3 constant domains. Unless otherwise specified herein, numbering of amino acid residues in the Fc region or constant region follows the EU numbering system, also referred to as the EU index.

The term "association" refers to a functional relationship between two or more polypeptide chains and/or two or more portions of a single polypeptide chain. In particular, the term "associate" means that two or more polypeptides (or parts of a single polypeptide) associate with each other, e.g., non-covalently through molecular interactions and/or covalently through one or more disulfide bridges or chemical cross-links, thereby generating a functional antigen binding domain. Examples of associations that may exist in an antigen-binding molecule include, but are not limited to, associations between Fc regions in an Fc domain, associations between VH and VL regions in an Fab or Fv, and associations between CH1 and CL in an Fab.

The term "Knob-into-Hole" refers to a modification for facilitating association of two polypeptide chains of an Fc, comprising a "knob" modification in one of the two polypeptide chains of an Fc and a "hole" modification in the other of the two polypeptide chains of an Fc. This technique is described, for example, in US 5,731,168 and US 7,695,936. Generally, the method involves introducing a protuberance ("knob") at the interface of the first polypeptide chain and a corresponding cavity ("hole") in the interface of the second polypeptide chain such that the protuberance can be positioned into the cavity to promote heterodimer formation and hinder homodimer formation. The protuberances are constructed by replacing small amino acid side chains from the first polypeptide chain interface with larger side chains, such as tyrosine or tryptophan. Complementary cavities of the same or similar size as the protuberances are created in the interface of the second polypeptide chain by replacing large amino acid side chains with smaller amino acid side chains (e.g. alanine or threonine).

Thus, in a particular embodiment, in the CH3 domain of the first polypeptide chain of the Fc domain of the bispecific antibody of the present disclosure, one amino acid residue is replaced with an amino acid residue having a larger side chain volume, thereby creating a protuberance within the CH3 domain of the first polypeptide chain that can be positioned in a cavity within the CH3 domain of the second polypeptide chain, and in the CH3 domain of the second polypeptide chain of the Fc domain, one amino acid residue is replaced with an amino acid residue having a smaller side chain volume, thereby creating a cavity within the CH3 domain of the second polypeptide chain in which the protuberance within the CH3 domain of the first polypeptide chain can be positioned. Preferably, the amino acid residue having a larger side chain volume is selected from the group consisting of arginine (R), phenylalanine (F), tyrosine (Y), and tryptophan (W). Preferably, the amino acid residue having a smaller side chain volume is selected from the group consisting of alanine (A), serine (S), threonine (T), and valine (V).

The term "linker" refers to any means used to connect two different functional units (e.g., antigen-binding fragments). Types of linkers include but are not limited to chemical linkers and polypeptide linkers. The sequence of the polypeptide linker is not limited. Polypeptide linkers are preferably non-immunogenic and flexible, such as those sequences comprising serine and glycine. Linkers can be long or short depending on the particular construct.

According to the present disclosure, the linker connecting different functional units preferably comprises a flexible peptide linker, such as a glycine-serine peptide linker. In one embodiment, the linker comprises an amino acid sequence $(G_4S)_n$ or $(G_4S)_nA$, wherein n is any integer selected from 1 to 10, preferably comprises an amino acid sequence $(G_4S)_3$ or $(G_4S)_3A$. The linker connecting the VH and VL domains to form the scFv domains of VH-VL or VL-VH preferably comprises a flexible peptide linker, such as a glycine-serine peptide linker. In one embodiment, the linker comprises an amino acid sequence $(G_4S)_n$ or $(G_4S)_nA$, wherein n is any integer selected from 1 to 10, preferably comprises an amino acid sequence $(G_4S)_3$ or $(G_4S)$.

As used herein, "antibody" may be used in the broadest sense and may include, for example, polyclonal antibodies, monoclonal antibodies, chimeric antibodies, humanized and primated antibodies, CDR-grafted antibodies, human antibodies (including recombinantly produced human antibodies), recombinantly produced antibodies, intracellular antibodies, multispecific antibodies, bispecific antibodies, monovalent antibodies, multivalent antibodies, anti-idiotype antibodies, synthetic antibodies (including mutant proteins and variants thereof), etc.

The term "monoclonal antibody" (or "mAb") refers to a substantially homogeneous antibody produced by a single cell clone that is only directed to a specific antigen epitope. Monoclonal antibodies can be prepared using a variety of techniques known in the art, including hybridoma techniques, recombinant techniques, phage display techniques, transgenic animals, synthetic techniques, or combinations of the above techniques, etc.

It should be noted that the CDRs and FRs of the variable regions of the antibodies and bispecific antigen-binding molecules of the present disclosure are defined according to Kabat definitions. Other naming and numbering systems, such as Chothia, IMGT or AHo, are known to those skilled in the art. Thus, humanized antibodies comprising one or more CDRs derived from any nomenclature system based on the monoclonal antibody sequences of the present disclosure are expressly maintained within the scope of the present disclosure.

The term "humanized antibody" refers to an antibody in which all or part of the amino acids other than CDRs of a non-human antibody (e.g., a mouse antibody) are replaced with corresponding amino acids derived from human immunoglobulin. Minor additions, deletions, insertions, substitutions or modifications of amino acids are permissible as long as they do not eliminate the ability of the antibody to bind to a specific antigen. "Humanized" antibodies retain antigen specificity similar to that of the original antibody.

The term "chimeric antibody" refers to an antibody in which the variable regions are derived from one species and the constant regions are derived from another species, for example, an antibody in which the variable regions are derived from a mouse antibody and the constant regions are derived from a human antibody.

The term "antibody fragment" encompasses at least a portion of an intact antibody. As used herein, a "fragment" of an antibody molecule includes an "antigen-binding fragment" of an antibody, and the term "antigen-binding fragment" refers to a polypeptide fragment of an immunoglobulin or antibody that specifically binds to or reacts with a selected antigen or immunogenic determining portion thereof, or a fusion protein product further derived therefrom, e.g. a single-chain antibody, an extracellular binding region in a chimeric antigen receptor, etc. Exemplary antibody fragments or antigen-binding fragments thereof include, but are not limited to, variable light chain fragments, variable heavy chain fragments, Fab fragments, $F(ab')_2$ fragments, Fd fragments, Fv fragments, single domain antibodies, linear antibodies, single chain antibodies (scFv), bispecific or multispecific antibodies formed from antibody fragments, etc.

The term "antigen" refers to a substance recognized and specifically bound by an antibody or antibody binding fragment, in a broad sense, an antigen may include any immunogenic fragment or determinant of a selected target, including a single epitope, multiple epitopes, a single domain, multiple domains, a complete extracellular domain (ECD) or a protein. Peptides, proteins, glycoproteins, polysaccharides and lipids, parts thereof and combinations thereof may also constitute antigens. Non-limiting exemplary antigens include tumor antigens or pathogen antigens, etc. "Antigen" may also refers to molecules that elicit an immune response. Any form of the antigen or cells or preparations containing the antigen can be used to generate antibodies specific for the antigenic determinant. The antigen may be an isolated full-length protein, a cell surface protein (e.g., immunized with cells expressing at least a portion of the antigen on their surface), or a soluble protein (e.g., immunized with only the ECD portion of the protein) or a protein construct (e.g., an Fc antigen). The antigen may be produced in genetically modified cells. Any of the foregoing antigens may be used alone or in combination with one or more immunogenicity enhancing adjuvants known in the art. The DNA encoding the antigen may be genomic or non-genomic (e.g., cDNA), and may encode at least a portion of the ECD sufficient to elicit an immunogenic response. Any vector may be used to transform cells in which the antigen is expressed, including but not limited to adenoviral vectors, lentiviral vectors, plasmids, and non-viral vectors such as cationic lipids.

The terms "epitope" and "antigenic determinant" refer to a site on an antigen to which an immunoglobulin or antibody specifically binds. Epitopes may be formed from adjacent amino acids or non-adjacent amino acids juxtaposed by tertiary folding of the protein. Epitopes formed by adjacent amino acids are generally retained after exposure to denaturing solvents, while epitopes formed by tertiary folding are generally lost after denaturing solvent treatment. Epitopes generally exist in unique spatial conformations and comprise at least 3-15 amino acids. Methods for determining the epitope bound by a given antibody are well known in the art and include immunoblotting and immunoprecipitation assays, etc. Methods for determining the spatial conformation of epitopes include techniques in the art, such as X-ray crystallography and two-dimensional nuclear magnetic resonance.

The term "bispecific" means that an antigen-binding molecule is capable of specifically binding to two different antigenic determinants. The term "antigen-binding molecule" in its broadest sense refers to a molecule that specifically binds to an antigenic determinant. Examples of antigen-binding molecules are immunoglobulins and derivatives thereof, e.g. fragments. The term "bispecific antigen-binding molecule " refers to a binding molecule (e.g. an antibody or a molecule comprising an antibody fragment) that has specificity for two different antigens (or epitopes), preferably a bispecific antibody.

The term "specifically binding" means that the binding is selective for the antigen and can be distinguished from unwanted or non-specific interactions. The ability of an antibody to bind to a specific antigenic determinant can be determined by an enzyme linked immunosorbent assay (ELISA) or other technique familiar to those skilled in the art, such as surface plasmon resonance (SPR) technique (analyzed on a BIAcore instrument).

When the variable regions in the present disclosure are used to produce antibodies, binding molecules, bispecific binding molecules or multispecific binding molecules, the constant regions are not particularly limited, and constant regions known to those skilled in the art or constant regions obtained by themselves can be used. Amino acid mutations (e.g., mutations that increase or decrease binding to Fcγ receptors or FcRn) can also be introduced into the constant region portion.

The method for obtaining the binding molecules, antigen-binding fragments, antibodies, bispecific binding molecules or multispecific binding molecules of the present disclosure is not particularly limited and can be obtained by any method, such as the Cold Spring Harbor Guide to Antibody Experimental Techniques, Chapters 5-8 and 15. Binding molecules, antigen-binding fragments, antibodies, bispecific binding molecules or multispecific binding molecules of the present disclosure can be prepared and purified using conventional methods. For example, cDNA sequences encoding heavy and light chains can be cloned and recombined into expression vectors. Recombinant immunoglobulin expression vector can be stably transfected into CHO cells. As a more preferred prior art, mammalian expression systems result in glycosylation of antibodies, particularly at the highly conserved N-terminal of the Fc region. Stable clones are obtained by expressing antibodies that specifically bind to human derived antigens. Positive clones are expanded in serum-free medium in bioreactors to produce antibodies. Antibody-secreting cultures can be purified and collected using conventional techniques. Antibodies can be filtered and concentrated by conventional methods. Soluble mixtures and polymers can also be removed by conventional methods, such as molecular sieves and ion exchange.

The term "antibody-drug conjugate" (ADC) refers to an antibody to which a therapeutically active substance or active pharmaceutical ingredient (API) has been covalently coupled, so that the therapeutically active substance or active pharmaceutical ingredient (API) can be targeted to the binding target of the antibody to exhibit its pharmacological function. The therapeutically active substance or active pharmaceutical ingredient may be a cytotoxin capable of killing cells targeted by the ADC, preferably malignant or cancerous cells. Covalent attachment of the therapeutically active substance, active pharmaceutical ingredient or cytotoxin may be performed in a non-site specific manner using standard chemical linkers coupling the payload to lysine or cysteine residues or, preferably, conjugation is performed in a site specific manner that allows complete control of the conjugation site and the drug-to-antibody ratio of the ADC produced.

The term "amino acid substitution" or "substitution" or " replacement" means replacing an amino acid at a specific position in a parent polypeptide sequence with another amino acid.

The term "affinity" or "binding affinity" refers to the strength of the sum of all non-covalent interactions between a single binding site of a molecule (e.g., an antibody) and its binding partner (e.g., an antigen). The term "KD" refers to the dissociation constant of a specific antibody-antigen interaction. Binding affinity can be determined using various techniques known in the art, such as surface plasmon resonance, bio-layer interferometry, dual polarization interferometry, static light scattering, dynamic light scattering, isothermal titration calorimetry, ELISA, analytical ultracentfugation and flow cytometry, etc.

The term "biological activity" refers to the ability of an antibody to bind to an antigen and cause a measurable biological response that can be measured in vitro or in vivo.

The pharmaceutical composition of the present disclosure may be formulated by mixing with appropriate pharmaceutically acceptable carriers, media, etc. that are inert thereto as required. For example: physiological saline, sterile water, excipients, stabilizers, antioxidants (such as ascorbic acid, etc.), buffers, preservatives, surfactants, chelating agents (such as EDTA, etc.) or adhesives, etc. In addition, it may also contain other low molecular weight polypeptides, proteins such as serum albumin, gelatin and immunoglobulin, amino acids such as glycine, glutamine, asparagine, glutamic acid, aspartic acid, methionine, arginine and lysine, sugars or carbohydrates such as polysaccharides and monosaccharides, and sugar alcohols such as mannitol and sorbitol. In the case of preparation as an aqueous solution for injection, examples include physiological saline, isotonic solutions containing glucose and other adjuvants, such as D-sorbitol, D-mannose, D-mannitol, and sodium chloride. It can also be used in combination with appropriate cosolvents, such as alcohols (ethanol, etc.), polyols (propylene glycol, PEG, etc.), non-ionic surfactants (polysorbate 80, polysorbate 20, poloxamer 188, HCO-50), etc. In addition, by mixing hyaluronidase into the preparation, subcutaneous administration of a larger liquid amount can also be performed.

The binding molecules or antigen-binding fragments of the present disclosure can be used in combination with other drugs, and the active ingredients can be mixed together to form a single administration unit, or can be independently formed into administration units and used separately.

The term "effective amount" refers to a dosage of a pharmaceutical formulation of an antibody or fragment of the present disclosure that produces a desired effect in a treated patient after administration to the patient in single or multiple doses. The effective amount can be readily determined by the attending physician as one skilled in the art by considering a variety of factors such as ethnic differences; weight, age, and health status; the particular disease involved; the severity of the disease; the response of the individual patient; the particular antibody administered; the pattern of administration; the bioavailability features of the administered formulation; the dosing regimen selected; and the use of any concomitant therapies. As used herein, the term "individual" or "subject" refers to any animal, such as a mammal or marsupial. Individuals of the present disclosure include, but are not limited to, humans, non-human primates (e.g., cynomolgus or rhesus macaques or other types of macaques), mice, pigs, horses, donkeys, cattle, sheep, rats, and poultry of any kind.

As used herein, the terms "disease" or "condition" or "disorder" and the like refer to any alteration or disorder that damages or interferes with the normal function of a cell, tissue or organ. For example, the term "disease" includes, but is not limited to, tumors, pathogen infection, autoimmune diseases, T cell dysfunction diseases, or immune tolerance defects (such as transplant rejection).

As used herein, the term "tumor" refers to a disease characterized by pathological proliferation of cells or tissues and their subsequent migration or invasion into other tissues or organs. Tumor growth is usually uncontrollable and progressive, without inducing or inhibiting normal cell proliferation.

As used herein, the term "treatment" refers to clinical intervention in an attempt to alter the disease process in an individual or cell-mediated manner, either for prevention or intervention during clinical pathology. The therapeutic effects include, but are not limited to, preventing the occurrence or recurrence of the disease, alleviating symptoms, reducing any direct or indirect pathological consequences of the disease, preventing metastasis, slowing the progression of the disease, improving or alleviating the condition, relieving or improving prognosis, etc.

## EMBODIMENT

The present disclosure is further described below in conjunction with specific examples. It should be understood that these examples are only used to illustrate the present disclosure but are not used to limit the scope of the present disclosure. The experimental methods in the following examples without specifying specific conditions are usually carried out under conventional conditions or conditions recommended by the manufacturers.

### Example 1. Design and sequences of anti-CD3-DLL3 bispecific antibodies

The bispecific antigen-binding molecules (hereinafter referred to as bispecific antibodies) constructed in the examples are formed by connecting anti-DLL3 nanobody or the heavy chain of anti-DLL3 full-length antibody Fab fragment and the binding domain of human T cell receptor subunit CD3$\epsilon$ via a flexible linker, wherein the anti-DLL3 nanobody is hDLL3-3-1-NA, and the sequence thereof is shown in SEQ ID NO: 5. The anti-DLL3 full-length antibody is H2-39E2D11-NA, the heavy chain sequence of which is shown in SEQ ID NO: 9, and the light chain sequence of which is shown in SEQ ID NO: 10. The CD3$\epsilon$ binding domain is derived from the full-length antibody h160C9AA, the heavy chain sequence of which is shown in SEQ ID NO: 19 and the light chain sequence of which is shown in SEQ ID NO: 20. In order to reduce ADCC activity of the antibody, amino acid substitutions L234A and L235A have been applied in the Fc region of the finally constructed bispecific antibodies.

The heavy chain variable region and the light chain variable region of h160C9AA are connected via a flexible linker to form a single-chain antibody scFv, the structure of which is VH-(G$_4$S)$_3$-VL, and the sequence is shown in SEQ ID NO: 23. The scFv is then fused with the C-terminal of the DLL3 full-length antibody Fab heavy chain or the DLL3 nanobody via a flexible linker.

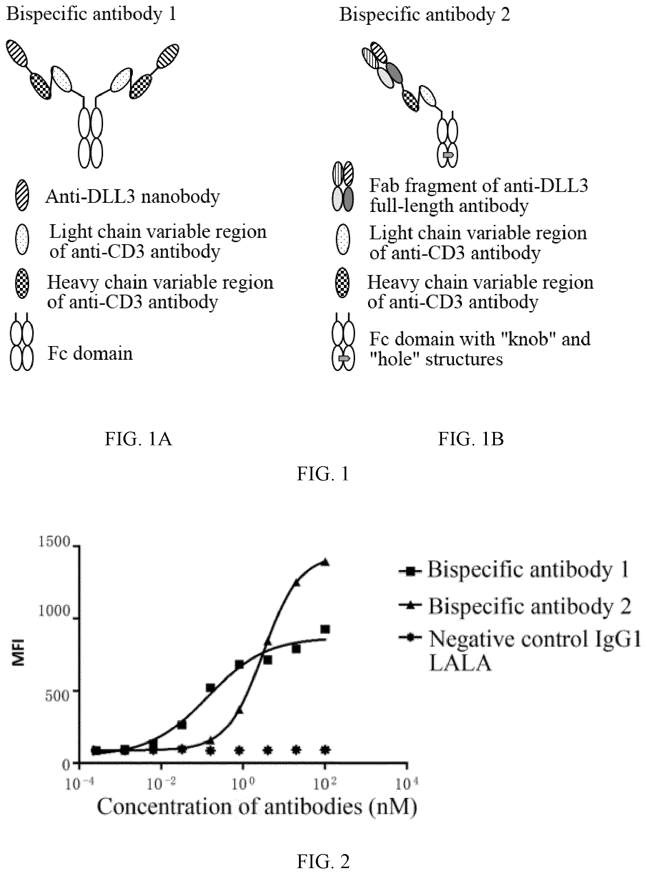

When scFv is fused with the C-terminal of the DLL3 nanobody, the formed bispecific antibody is named Bispecific antibody 1 and comprises two homologous chains, the sequence of the fused chain is shown in SEQ ID NO: 1, schematically shown in FIG. 1A.

When scFv is fused with the C-terminal end of the heavy chain of the Fab fragment of the DLL3 full-length antibody, the formed bispecific antibody is named Bispecific antibody 2 and comprises one light chain (i.e., the third polypeptide) and two heterologous heavy chains (i.e., the first polypeptide and the second polypeptide). Among the two heterologous heavy chains, the heavy chain containing scFv was designed as a "knob" structure (named Heavy chain with "knob" structure of Bispecific antibody 2), including amino acid substitutions at S354C and T366W. Among the two heterologous heavy chains, the heavy chain that does not contain scFv is designed as a "hole" structure (named Heavy chain with "hole" structure of Bispecific antibody 2), including amino acid substitutions at Y349C, T366S, L368A and Y407V. In addition, in order to facilitate the purification of bispecific antibodies, the heavy chain of the "hole" structure should also be applied with H435R substitution. The sequence of the heavy chain of the modified "knob" structure is shown in SEQ ID NO: 2, the sequence of the heavy chain of the "hole" structure is shown in SEQ ID NO: 3, and the sequence of the light chain is shown in SEQ ID NO: 4 (named as the Light chain of the Bispecific antibody 2). The schematic diagram is shown in FIG. 1B.

The structures and related molecular sequences of Bispecific antibodies 1 and 2 are summarized in Table 1 and Table 2, respectively.

Table 1 Structure of bispecific antibodies

| Name of molecule | | Molecular structure description | SEQ ID NO: |
|---|---|---|---|
| Bispecific antibody 1 | | DLL3 VHH - (G$_4$S) - CD3VH - (G$_4$S)$_3$ - CD3VL - (EPKSS) - IgG1 Fc | 1 |
| Bispecific antibody 2 | Heavy chain with "knob" structure | DLL3 VH-DLL3 CH1-(G$_4$S)$_3$-CD3VH- (G$_4$S)$_3$-CD3VL-(EPKSS)-IgG1 Fc (knob) | 2 |
| | Heavy chain with "hole" structure | IgG1 Fc (hole) | 3 |
| | Light chain | DLL3 VL - DLL3 CL (i.e. DLL3 LC) | 4 |
| hDLL3-3-1-NA | | DLL3 VHH - IgG1 Fc | 5 |
| H2-39E2D11-NA | | DLL3 HC | 9 |
| | | DLL3 LC | 10 |
| h160C9AA | | CD3 HC | 19 |
| | | CD3 LC | 20 |
| h160C9AA scFv | | CD3VH - (G$_4$S)$_3$ - CD3VL | 23 |

Table 2 Amino acid sequences of bispecific antibodies

| Name | SEQ ID NO: | Amino acid sequence |
|---|---|---|
| Bispecific antibody 1 | 1 | EVQLVESGGGLVQPGGSLRLSCAASTYTISSGYMGW FRQAPGKEREGVAAIYIGGSTTLYADSVKGRFTISRDN SKNTLYLQMNSLRAEDTAVYYCAAQLRPNAAYHPLD GRKYNYWGQGTLVTVSSGGGGSEVKLVESGGGLVQ PGGSLKLSCAASGFTFNTYAMNWVRQAPGKGLEWV ARIRSKYNNYATYYADSVKDRFTISRDDAKNTLYLQ MNNLRTEDTAVYYCVRHGNFGNSYVSWFAYWGQGT LVTVSSGGGGSGGGGSGGGGSQAVVTQEPSLTVSPG GTVTLTCRSSTGAVTTSNYANWVQQKPGQAPRGLIG GTNKRAPGTPARFSGSLLGGKAALTLSGAQPEDEAEY YCALWYSNLWVFGGGTKLTVLEPKSSDKTHTCPPCP APEAAGGPSVFLFPPKPKDTLMISRTPEVTCVVVDVS HEDPEVKFNWYVDGVEVHNAKTKPREEQYNSTYRV VSVLTVLHQDWLNGKEYKCKVSNKALPAPIEKTISKA KGQPREPQVYTLPPSRDELTKNQVSLTCLVKGFYPSDI AVEWESNGQPENNYKTTPPVLDSDGSFFLYSKLTVDK SRWQQGNVFSCSVMHEALHNHYTQKSLSLSPGK |
| Heavy chain with "knob" structure of Bispecific antibody 2 | 2 | QVQLVQSGAEVKKPGASVKVSCKASGYTFISYWITW VRQAPGQGLEWMGDIYPGSGSTTNYNEKFKSRVTMT RDTSTSTVYMELSSLRSEDTAVYYCARETTVGGAYA MDYWGQGTLVTVSSASTKGPSVFPLAPSSKSTSGGTA ALGCLVKDYFPEPVTVSWNSGALTSGVHTFPAVLQSS GLYSLSSVVTVPSSSLGTQTYICNVNHKPSNTKVDKK VEPKSCGGGGSGGGGSGGGGSEVKLVESGGGLVQPG GSLKLSCAASGFTFNTYAMNWVRQAPGKGLEWVARI RSKYNNYATYYADSVKDRFTISRDDAKNTLYLQMNN LRTEDTAVYYCVRHGNFGNSYVSWFAYWGQGTLVT VSSGGGGSGGGGSGGGGSQAVVTQEPSLTVSPGGTV TLTCRSSTGAVTTSNYANWVQQKPGQAPRGLIGGTN KRAPGTPARFSGSLLGGKAALTLSGAQPEDEAEYYC ALWYSNLWVFGGGTKLTVLEPKSSDKTHTCPPCPAPE AAGGPSVFLFPPKPKDTLMISRTPEVTCVVVDVSHED PEVKFNWYVDGVEVHNAKTKPREEQYNSTYRVVSV LTVLHQDWLNGKEYKCKVSNKALPAPIEKTISKAKG QPREPQVYTLPPCRDELTKNQVSLWCLVKGFYPSDIA VEWESNGQPENNYKTTPPVLDSDGSFFLYSKLTVDKS RWQQGNVFSCSVMHEALHNHYTQKSLSLSPGK |

(continued)

| Name | SEQ ID NO: | Amino acid sequence |
|---|---|---|
| Heavy chain with "hole" structure of Bispecific antibody 2 | 3 | DKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSRDELTKNQVSLSCAVKGFYPSDIAVEWESNGQPENNYKTTPPVLDSDGSFFLVSKLTVDKSRWQQGNVFSCSVMHEALHNRYTQKSLSLSPGK |
| Light chain of Bispecific antibody 2 | 4 | EIVLTQSPATLSLSPGERATLSCRASQSINNNLHWYQQKPGQAPRLLIKYVSQSISGIPARFSGSGSGTDFTLTISSLEPEDFAVYYCQQTNAWPLTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| hDLL3-3-1-NA Nanobody | 5 | EVQLVESGGGLVQPGGSLRLSCAASTYTISSGYMGWFRQAPGKEREGVAAIYIGGSTTLYADSVKGRFTISRDNSKNTLYLQMNSLRAEDTAVYYCAAQLRPNAAYHPLDGRKYNYWGQGTLVTVSSGGGGSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |
| hDLL3-3-1-NA Nanobody HCDR1 | 6 | SGYMG |
| hDLL3-3-1-NA Nanobody HCDR2 | 7 | AAIYIGGSTTLYADSVKG |
| hDLL3-3-1-NA Nanobody HCDR3 | 8 | QLRPNAAYHPLDGRKYNY |
| H2-39E2D11-NA Heavy Chain | 9 | QVQLVQSGAEVKKPGASVKVSCKASGYTFISYWITWVRQAPGQGLEWMGDIYPGSGSTTNYNEKFKSRVTMTRDTSTSTVYMELSSLRSEDTAVYYCARETTVGGAYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| Name | SEQ ID NO: | Amino acid sequence |
|---|---|---|
| H2-39E2D11-NA Light Chain | 10 | EIVLTQSPATLSLSPGERATLSCRASQSINNNLHWYQQ KPGQAPRLLIKYVSQSISGIPARFSGSGSGTDFTLTISSL EPEDFAVYYCQQTNAWPLTFGGGTKLEIKRTVAAPSV FIFPPSDEQLKSGTASVVCLLNNFYPREAKVQWKVDN ALQSGNSQESVTEQDSKDSTYSLSSTLTLSKADYEKH KVYACEVTHQGLSSPVTKSFNRGEC |
| H2-39E2D11-NA heavy chain variable region VH | 11 | QVQLVQSGAEVKKPGASVKVSCKASGYTFISYWITW VRQAPGQGLEWMGDIYPGSGSTTNYNEKFKSRVTMT RDTSTSTVYMELSSLRSEDTAVYYCARETTVGGAYA MDYWGQGTLVTVSS |
| H2-39E2D11-NA light chain variable region VL | 12 | EIVLTQSPATLSLSPGERATLSCRASQSINNNLHWYQQ KPGQAPRLLIKYVSQSISGIPARFSGSGSGTDFTLTISSL EPEDFAVYYCQQTNAWPLTFGGGTKLEIK |
| H2-39E2D11-NA Heavy Chain HCDR1 | 13 | SYWIT |
| H2-39E2D11-NA Heavy Chain HCDR2 | 14 | DIYPGSGSTTNYNEKFKS |
| H2-39E2D11-NA Heavy Chain HCDR3 | 15 | ETTVGGAYAMDY |
| H2-39E2D11-NA Light Chain LCDR1 | 16 | RASQSINNNLH |
| H2-39E2D11-NA Light Chain LCDR2 | 17 | YVSQSIS |
| H2-39E2D11-NA Light Chain LCDR3 | 18 | QQTNAWPLT |
| h160C9AA Heavy Chain | 19 | EVKLVESGGGLVQPGGSLKLSCAASGFTFNTYAMNW VRQAPGKGLEWVARIRSKYNNYATYYADSVKDRFTI SRDDAKNTLYLQMNNLRTEDTAVYYCVRHGNFGNS YVSWFAYWGQGTLVTVSSASTKGPSVFPLAPSSKSTS GGTAALGCLVKDYFPEPVTVSWNSGALTSGVHTFPAV LQSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSRDE LTKNQVSLTCLVKGFYPSDIAVEWESNGQPENNYKTT PPVLDSDGSFFLYSKLTVDKSRWQQGNVFSCSVMHE ALHNHYTQKSLSLSPGK |

(continued)

| Name | SEQ ID NO: | Amino acid sequence |
|---|---|---|
| h160C9AA Light Chain | 20 | QAVVTQEPSLTVSPGGTVTLTCRSSTGAVTTSNYANW VQQKPGQAPRGLIGGTNKRAPGTPARFSGSLLGGKA ALTLSGAQPEDEAEYYCALWYSNLWVFGGGTKLTVL GQPKANPTVTLFPPSSEELQANKATLVCLISDFYPGAV TVAWKADSSPVKAGVETTTPSKQSNNKYAASSYLSLT PEQWKSHRSYSCQVTHEGSTVEKTVAPTEC |
| h160C9AA heavy chain variable region VH | 21 | EVKLVESGGGLVQPGGSLKLSCAASGFTFNTYAMN WVRQAPGKGLEWVARIRSKYNNYATYYADSVKDRF TISRDDAKNTLYLQMNNLRTEDTAVYYCVRHGNFG NSYVSWFAYWGQGTLVTVSS |
| h160C9AA light chain variable region VL | 22 | QAVVTQEPSLTVSPGGTVTLTCRSSTGAVTTSNYAN WVQQKPGQAPRGLIGGTNKRAPGTPARFSGSLLGGK AALTLSGAQPEDEAEYYCALWYSNLWVFGGGTKLT VL |
| scFv of h160C9AA | 23 | EVKLVESGGGLVQPGGSLKLSCAASGFTFNTYAMN WVRQAPGKGLEWVARIRSKYNNYATYYADSVKDRF TISRDDAKNTLYLQMNNLRTEDTAVYYCVRHGNFG NSYVSWFAYWGQGTLVTVSSGGGGSGGGGSGGGGS QAVVTQEPSLTVSPGGTVTLTCRSSTGAVTTSNYAN WVQQKPGQAPRGLIGGTNKRAPGTPARFSGSLLGGK AALTLSGAQPEDEAEYYCALWYSNLWVFGGGTKLT VL |
| h160C9AA Heavy Chain HCDR1 | 24 | TYAMN |
| h160C9AA Heavy Chain HCDR2 | 25 | RIRSKYNNYATYYADSVKD |
| h160C9AA Heavy Chain HCDR3 | 26 | HGNFGNSYVSWFAY |
| h160C9AA Light Chain LCDR1 | 27 | RSSTGAVTTSNYAN |
| h160C9AA Light Chain LCDR2 | 28 | GTNKRAP |
| h160C9AA Light Chain LCDR3 | 29 | ALWYSNLWV |
| Fc in Bispecific antibody 1 | 30 | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQK SLSLSPGK |

(continued)

| Name | SEQ ID NO: | Amino acid sequence |
|---|---|---|
| Fc with "knob" structure in Bispecific antibody 2 | 31 | DKTHTCPPCPAPEAAGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPCRDELTKNQVSLW CLVKGFYPSDIAVEWESNGQPENNYKTTPPVLDSDGS FFLYSKLTVDKSRWQQGNVFSCSVMHEALHNHYTQK SLSLSPGK |
| VHH in Bispecific antibody 1 | 32 | EVQLVESGGGLVQPGGSLRLSCAASTYTISSGYMGW FRQAPGKEREGVAAIYIGGSTTLYADSVKGRFTISRDN SKNTLYLQMNSLRAEDTAVYYCAAQLRPNAAYHPLD GRKYNYWGQGTLVTVSS |
| Fab heavy chain in Bispecific antibody 2 | 33 | QVQLVQSGAEVKKPGASVKVSCKASGYTFISYWITW VRQAPGQGLEWMGDIYPGSGSTTNYNEKFKSRVTMT RDTSTSTVYMELSSLRSEDTAVYYCARETTVGGAYA MDYWGQGTLVTVSSASTKGPSVFPLAPSSKSTSGGTA ALGCLVKDYFPEPVTVSWNSGALTSGVHTFPAVLQSS GLYSLSSVVTVPSSSLGTQTYICNVNHKPSNTKVDKK VEPKSC |

## Example 2. Construction of anti-CD3-DLL3 bispecific antibodies and transient transfection expression thereof in eukaryotic cells

Gene fragments of aforementioned bispecific antibody molecules were cloned into PTT5 expression vectors respectively to prepare transfection level expression plasmids.

Expi 293F™ cells (Thermo Fisher Scientific) were cultured in serum-free medium and the cells were seeded in shake flasks (Corning Inc.) and incubated on a shaker at 37°C and 8% $CO_2$. After adjusting cell density, the recombinant expression vector containing the target gene fragment and PEI transfection reagent were mixed according to a suitable ratio, and added into cell culture shake flask. After cell culture for 6 days, the expression supernatant was collected, centrifuged at high speed to remove cell debris, and affinity purified by Protein A column. Rinse the column with PBS until the A280 reading drops to baseline. The target protein was eluted with pH 3.0-pH 3.5 acidic eluant and neutralized with 1M Tris-HCl, pH 8.0-9.0. After appropriate concentration of the eluted sample, the sample was further purified by PBS equilibrated gel chromatography Superdex200 (GE) to remove aggregates, collect monomer peaks, and exchange the solution to PBS for aliquoting. SDS-PAGE and HPLC purity analysis and A280 concentration determination were performed on the final purified antibodies.

## Example 3. Affinity test of anti-CD3-DLL3 bispecific antibodies

A. Affinity test of anti-CD3-DLL3 bispecific antibodies to cells expressing hDLL3 and hCD3

FACS was used to detect the binding of anti-CD3-DLL3 bispecific antibodies to SHP-77 cells expressing hDLL3 and T lymphocytes (Jurkat) naturally expressing hCD3.

SHP-77 cells (ATCC, CRL-2195) and Jurkat cells (ATCC, TIB-152) were cultured in RPMI 1640 + 10% FBS medium in T75 cell culture flasks at 37 °C in 5% $CO_2$ incubator. When the cells were ready for use, the SHP-77 cells were washed with sterile DPBS, digested with 0.25% trypsin EDTA for about 5 minutes, and then terminated with complete culture medium and placed in a 50 mL centrifuge tube. Jurkat cells were placed directly into 50 mL centrifuge tubes without digestion.

SHP-77 and Jurkat were centrifuged at 1000 rpm for 5 minutes at room temperature, the supernatant was discarded, and the cells were resuspended with 100 μL 1% BSA (in PBS). Cells were counted and cell density was adjusted to 1E6/mL. The cells were plated into 96-well round-bottom culture plates (corning, Cat. No.3799), centrifuged at 1500 rpm for 5 minutes at 4°C, and the supernatant was discarded and the plate was stored at 4°C for later use. 1% BSA (in PBS) was used to dilute the test antibody and negative control IgG1 LALA (purchased from Biointron, Cat. No. B109802) at a starting concentration of 100 nM and 7 concentrations diluted 10-fold downward. The cells were resuspended with the diluted antibody, 100 μL/well, and incubated at 4°C for 1 hour. The cells were centrifuged at 1500 rpm at 4°C for 5 minutes and the supernatant was discarded, then resuspended and washed with 160 μL 1% BSA (in PBS), centrifuged at 1500 rpm at 4°C for 5 minutes, and the supernatant was discarded. The secondary antibody (goat anti human IgG Fc PE) was diluted at 1:200 with 1% BSA (in PBS) according to the instructions, the cells were resuspended with the diluted secondary antibody, 100 μL/well, and incubated at 4°C for 0.5 hours. The cells were centrifuged at 1500 rpm at 4°C for 5 minutes and the supernatant was discarded, then resuspend and washed with 160 μL 1% BSA (in PBS), centrifuged at 1500 rpm at 4°C for 5 minutes, and the supernatant was discarded. The cells were resuspended in 100 μL 1% BSA (in PBS), filtered through 300-mesh gauze, and the mean fluorescence intensity of the PE channel was detected by flow cytometry.

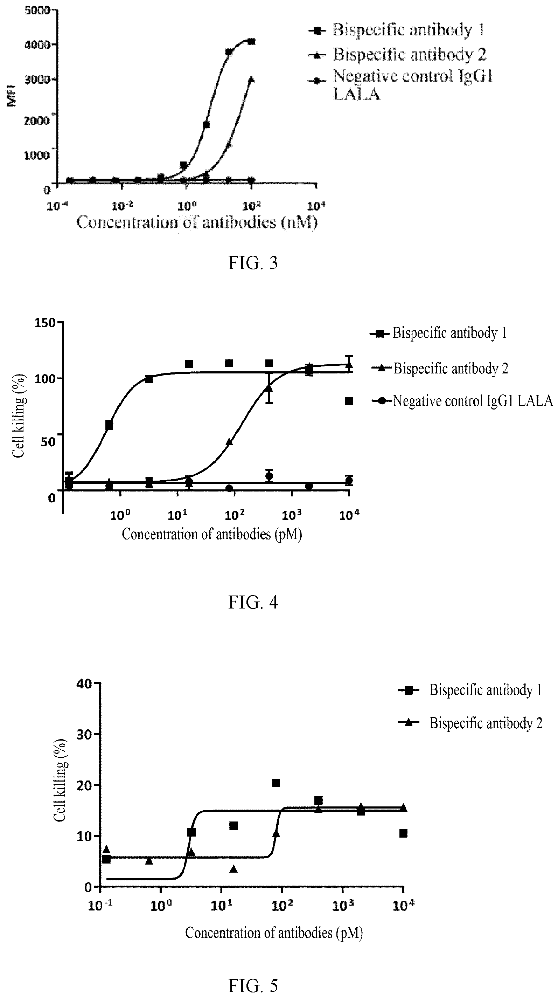

The FCS file was exported from the flow cytometer, and the PE channel mean fluorescence intensity (hereinafter referred to as MFI) of each sample was analyzed by flowjo software. The mean fluorescence intensity obtained by the analysis was imported into Graphpad to analyze the half-binding concentration of the antibody and cells (hereinafter referred to as $EC_{50}$). The results are shown in Table 3, FIG 2 (SHP-77 cells) and FIG 3 (Jurkat cells). The binding abilities of the two bispecific antibodies to the Jurkat cell line were weaker than those to the SHP-77 cell line, and the binding abilities of the Bispecific antibody 2 molecule to both cell lines were weaker than those of the Bispecific antibody 1 molecule.

Table 3 Affinity of bispecific antibodies to hDLL3 (SHP-77 cell line) and hCD3 (Jurkat cell line), respectively

| Molecule number | SHP-77 cell line | Jurkat cell line |
|---|---|---|
| | $EC_{50}$ (nM) | $EC_{50}$ (nM) |
| Bispecific antibody 1 | 0.14 | 5.28 |
| Bispecific antibody 2 | 3.11 | 57.92 |

B. Recombinant protein binding affinity and kinetics of anti-CD3-DLL3 bispecific antibodies in vitro

The affinity and kinetic properties of anti-CD3-DLL3 bispecific antibody molecules to human/cynomolgus DLL3 and human/cynomolgus CD3 were analyzed using a Biacore 8K instrument.

To determine the affinity and kinetic properties of human DLL3 (purchased from Kaixia, Cat. No DLL-HM103) / cynomolgus DLL3 (purchased from Kaixia, Cat.No. DLL-RM103), the CM5 chip was first activated with EDC and NHS, then anti-human Fc mouse monoclonal antibody was fixed and then blocked with ethanolamine. The anti-CD3-DLL3 bispecific antibody molecule was diluted to 0.5 μg/mL with HBS-EP+ (10 mM HEPES, pH 7.4, 150 mM NaCl, 3 mM EDTA, 0.05% P20) buffer and captured at a flow rate of 10 μL/min for 45 s. Human/cynomolgus DLL3 was two-fold serial diluted to a series of concentrations (100 nM-0.78 nM) and bound for 90 s and dissociated for 450 s at a flow rate of 50 μL/min.

After each round of experiments, the chip was regenerated by flushing with 3M $MgCl_2$ solution at a flow rate of 30 μL/min for 30 s to remove the captured antibodies together with the antigens. The raw data were analyzed using Biacore Insight Evaluation Software (3.0.12.15655) and fitted with a (1:1) Langmuir model. The obtained bispecific antibody affinity and kinetic experimental data are shown in Table 4.

Table 4 Binding affinity and kinetics of anti-CD3-DLL3 bispecific antibodies to human/ cynomolgus DLL3 protein

| Molecule number | Antigen | Binding rate $k_a$ (1/M*s) | Dissociation rate $k_d$ (1/s) | Affinity $K_D$ (M) |
|---|---|---|---|---|
| Bispecific antibody 1 | Human DLL3 | 1.03E+06 | 1.64E-04 | 1.59E-10 |
| Bispecific antibody 2 | Human DLL3 | 1.22E+05 | 7.92E-05 | 6.47E-10 |
| Bispecific antibody 1 | Cynomolgus DLL3 | 3.19E+05 | 1.32E-03 | 4.15E-09 |
| Bispecific antibody 2 | Cynomolgus DLL3 | 9.70E+04 | 8.60E-05 | 8.87E-10 |

In order to determine the affinity and kinetic properties of human CD3 (purchased from Aero, Cat. No.CDD-H52W1) / cynomolgus CD3 (purchased from Aero, Cat. No.CDD-C52W4), a method for direct immobilization of human/cynomolgus CD3 molecules on CM5 chips was used. CM5 chips were activated with EDC and NHS, CD3 molecules from human/cynomolgus were diluted to 1 μg/ml with acetate solution at pH 5, solidified at a flow rate of 10 μL/min for 60 s, and then blocked with ethanolamine. Anti-CD3-DLL3 bispecific antibody molecules were two-fold serial diluted to a series of concentrations (100 nM-0.39 nM) with HBS-EP+ (10 mM HEPES, pH 7.4, 150 mM NaCl, 3 mM EDTA, 0.05% P20) buffer, bound at a flow rate of 50 μL/min for 90 s, and dissociated for 360 s.

After each round of experiments, the chip was regenerated by flushing with 3M MgCl$_2$ solution at a flow rate of 30 μL/min for 30 s to remove anti-CD3-DLL3 bispecific antibody molecules. The raw data were analyzed using Biacore Insight Evaluation Software (3.0.12.15655) and fitted with a (1:1) Langmuir model. The obtained bispecific antibody affinity and kinetic experimental data are shown in Table 5.

Table 5 Binding affinity and kinetics of anti-CD3-DLL3 bispecific antibody to human/ cynomolgus CD3 protein

| Molecule number | Antigen | Binding rate $k_a$ (1/m*s) | Dissociation rate $K_d$ (1/s) | Affinity $K_d$(m) |
|---|---|---|---|---|
| Bispecific antibody 1 | Human CD3 | 3.19E+06 | 7.84E-04 | 2.46E-10 |
| Bispecific antibody 2 | Human CD3 | 4.56E+05 | 4.14E-03 | 9.09E-09 |
| Bispecific antibody 1 | Cynomolgus CD3 | 2.54E+06 | 6.11E-04 | 2.40E-10 |
| Bispecific antibody 2 | Cynomolgus CD3 | 3.65E+05 | 4.05E-03 | 1.11E-08 |

The experimental results showed that the two bispecific antibody molecules bound to both human/cynomolgus DLL3 and human/cynomolgus CD3, and the affinity of CD3 terminal of Bispecific antibody 1 molecule was stronger than DLL3 terminal or equivalent to DLL3 terminal; the affinity of DLL3 terminal of Bispecific antibody 2 molecule was stronger than CD3 terminal.

## Example 4. In vitro functional assay of anti-CD3-DLL3 bispecific antibodies

### A. T cell dependent cellular cytotoxicity assay (TDCC), target cells are SHP-77

SHP-77 cells were cultured in RPMI 1640+10% FBS culture medium in a T75 cell culture flask placed in a 37°C 5% CO$_2$ incubator. When the cells were to be used, they were washed with sterile DPBS, digested with 0.25% trypsin EDTA for about 5 minutes, and then terminated with complete culture medium. The cells were placed in a 50 mL centrifuge tube and centrifuged at 1000 rpm for 5 minutes. The supernatant was discarded, the cells were resuspended with complete medium and counted, and the cell density was adjusted to 5E4/mL.

The target cells were plated into a greiner black bottom permeable and wall impermeable 96-well plate (Cat. No. 655090), 100 μL/well, and cultured overnight at 37°C and 5% CO$_2$. CD3$^+$T cells were sorted from fresh PBMCs using a T cell negative selection kit (StemCell, Cat. No. 17951), the cells were counted and the cell density was adjusted to 5E5/mL using RPMI 1640+10% FBS. The effector cells were plated into a 96-well plate, 100 μL/well, and cultured at 37°C in 5% CO$_2$.

Antibodies were diluted with complete medium, starting at a concentration of 110 nM (11X), and diluted 5-fold downward. Diluted antibody was added to the cell culture plate at 20 μL/well, so the initial and final concentration is 10 nM. The cells were incubated at 37°C, 5% CO$_2$ for 48 hours. The lymphocytes in the plate were aspirated with a pipette tip, 100 μL CTG detection reagent was added to each well, and the plates were incubated at 300 rpm in the dark for 10 minutes, and the chemiluminescence was read on Envision.

The percentage of cell killing caused by the TDCC effect was calculated using the following formula:

$$\text{Cell killing \%} = 1 - (\text{Sample well value} - \text{T cell well value}) / (\text{Maximum signal value} - \text{T cell well value})$$

The T cell well values are the well values with only T cells added but no target cell SHP-77.

GraphPad software was used to calculate EC$_{50}$ and maximum killing, and the results are shown in Table 6 and FIG. 4. The maximum killing ability of the two Bispecific antibodies to SHP-77 cells reached 100%, and the half effective concentration of Bispecific antibody 1 was lower than that of Bispecific antibody 2.

### B. T cell dependent cellular cytotoxicity assay (TDCC), target cells are NCI-H82

NCI-H82 cells (ATCC, HTB-175) were cultured in RPMI 1640+10% FBS culture medium. T75 cell culture flasks were used and placed in a 37°C 5% CO$_2$ incubator for culturing. The cells were centrifuged at 1000 rpm for 5 minutes and resuspended in DPBS. Cells were counted and adjusted to a cell density of 1E6/ml, added with 30 nM Cell Trace Far Red Cell Proliferation Kit, and cultured and stained for 20 minutes in a 37°C 5% CO$_2$ incubator. An equal volume of complete medium was added, centrifuged at 1000 rpm for 5 minutes, the supernatant was discarded, and the cells were washed once with DPBS.

Target cells were resuspended in complete medium, counted, adjusted to a cell density of 2E5/mL and plated in round bottom plates (corning, Cat. No. 3799) at 50 $\mu$L/well. CD3$^+$ T cells were sorted from fresh PBMCs using a T cell negative selection kit (StemCell, Cat. No. 17951), counted and adjusted to a cell density of 2E6/mL with RPMI 1640+ 10% FBS. Effector cells were plated in 96-well plates, 100 $\mu$L/well, and incubated at 37°C with 5% $CO_2$.

Antibody dilution and sample addition: Antibodies were diluted with complete medium, starting at a concentration of 110 nM (11X), and diluted 5-fold downward. Diluted antibody was added to the cell culture plate at 10 $\mu$L/well, so the initial and final concentration is 10 nM. The cells were incubated at 37°C, 5% $CO_2$ for 48 hours. 10 $\mu$L of the live-death dye PI was added to each well, with a final concentration of PI of 4 $\mu$g/mL, mixed, and the cells were stained at room temperature for 10 minutes. The cells were filtered through 300-mesh gauze, and the ratios of APC channel (stained target cells) and PE channel (stained dead or alive) were detected by flow cytometry.

The percentage of cell killing caused by the TDCC effect was calculated using the following formula:

$$\text{Target cell death } \% = (\text{Far Red} + \text{PI} + \text{cells}) / [(\text{Far Red} + \text{PI} + \text{cells}) + (\text{Far Red} + \text{PI} - \text{cells})]$$

$$\text{Cell killing } \% = \text{death of target cells } \% - \text{spontaneous death of target cells } \%$$

The spontaneous death percentage of target cells is the number of dead cells in the wells with only target cells (NCI-H82) but no T cells.

GraphPad software was used to calculate $EC_{50}$ and maximum killing, and the results are shown in Table 6 and FIG. 5. It is reported in literature that the expression of DLL3 in NCI-H82 cells is only 1/3 of that in SHP-77 cells, so the maximum killing ability of two bispecific antibody molecules to NCI-H82 cells is much weaker than that of SHP-77 cells, both are about 15%, and the half effective concentration of Bispecific antibody 1 molecule is lower than that of Bispecific antibody 2 molecule.

Table 6 T cell dependent cellular cytotoxicity assay (TDCC) results of anti-CD3-DLL3 bispecific antibodies

| Molecule number | SHP-77 cell line | | NCI-H82 cell line | |
|---|---|---|---|---|
| | $EC_{50}$ (nM) | Maximum killing (%) | $EC_{50}$ (nM) | Maximum killing (%) |
| Bispecific antibody 1 | 0.58 | 100 | 2.94 | 15 |
| Bispecific antibody 2 | 135.7 | 100 | 80.16 | 15 |

C. Cytokine release assay

SHP-77 cells were cultured in RPMI 1640+ 10% FBS medium in T75 cell culture flasks at 37 °C in 5% $CO_2$ incubator. When the cells were to be used, they were washed with sterile DPBS, digested with 0.25% trypsin EDTA for about 5 minutes, and then terminated with complete culture medium. The cells were placed in a 50 mL centrifuge tube and centrifuged at 1000 rpm for 5 minutes. The supernatant was discarded, the cells were resuspended and counted in complete medium, and the cell density was adjusted to 1E5/mL. SHP-77 cells were plated into a 96-well plate (corning 3799) at 100 $\mu$L/well and cultured at 37°C with 5% $CO_2$.

Fresh PBMCs were purchased, cell counting was performed, and cell density was adjusted to 2E6/mL with RPMI 1640+ 10% FBS. Effector cells were plated in 96-well plates at 100 $\mu$L/ well and incubated at 37 °C with 5% $CO_2$.

Antibody was diluted in complete medium starting at 110 nM (11X) and diluted 5-fold downward. Diluted antibody was added to the cell culture plate at 20 $\mu$L/ well, so the initial final concentration was 10 nM. The cells were incubated at 37 °C 5% $CO_2$ for 48 hours.

The culture plate was centrifuged at 400 g for 10 minutes, and 80 $\mu$L of the supernatant was frozen and stored at -80°C for later use.

The cytokine standard was diluted according to the instructions of the CBA flow cytometry kit (BD, 551809), two-fold diluted, with the highest concentration being 5000 pg/mL and the lowest concentration being 20 pg/mL. The beads were vortexed and mixed, human Th1/Th2 cytokine capture beads were mixed in a 1:1 ratio, six cytokine beads were mixed in equal proportions, then added to a 96-well plate (corning 3799), 40 $\mu$L/well. The prepared standards or thawed samples were added to a 96-well plate at 40 $\mu$L/ well. Human Th1/Th2-II PE assay reagent was added to a 96-well plate, shaken at 1100 rpm for 5 minutes in the dark, and incubated at room temperature for 3 hours in the dark. The plate was washed with wash buffer, 160 $\mu$L/ well, twice. The samples were filtered through 300 mesh gauze, and the fluorescence readings of APC channel and PE channel were detected by flow cytometry.

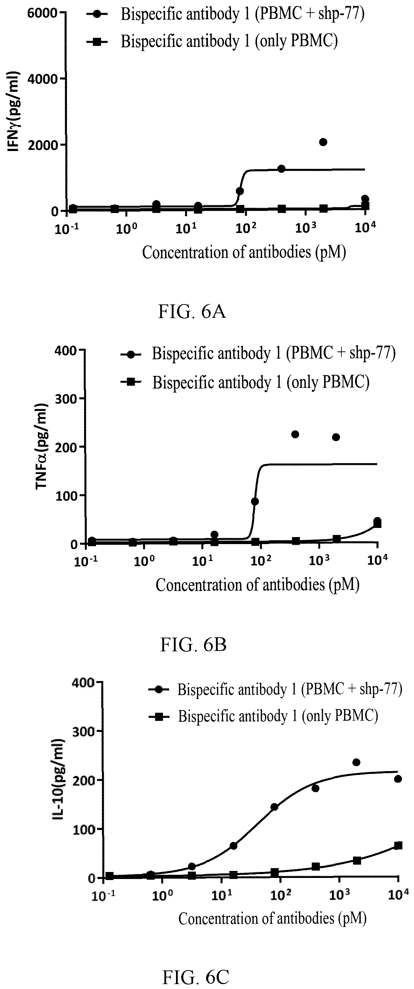

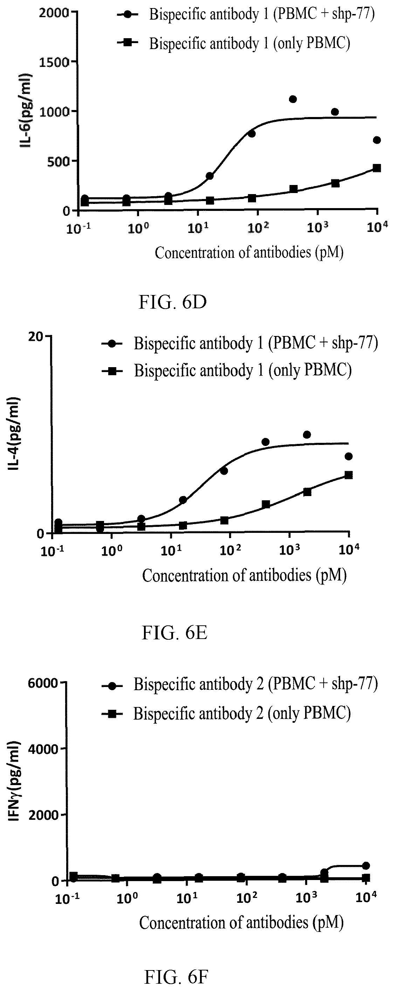

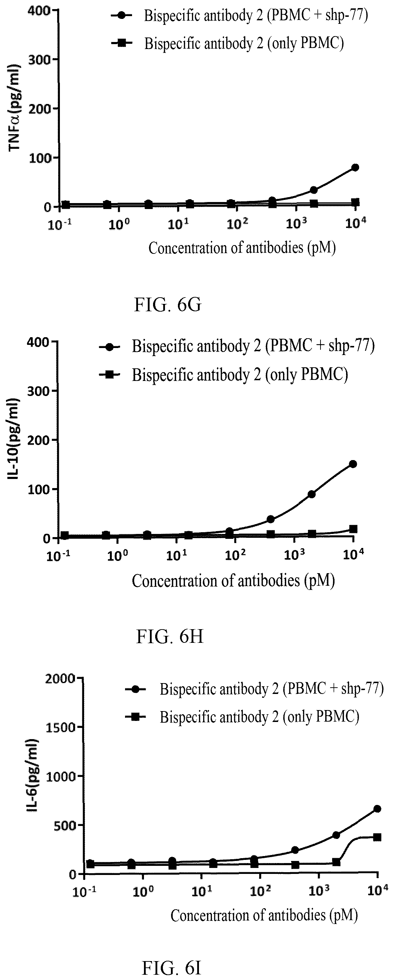

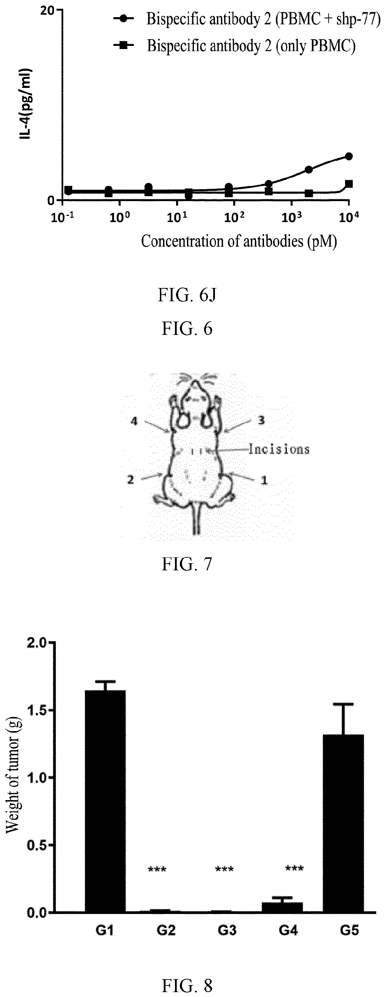

The FCS file was exported from the instrument and imported into FCAP software to analyze the cytokine release of each sample. The results are shown in FIG. 6 (FIG. 6A-6E show the cytokine release results of IFNy, TNF$\alpha$, IL-10, IL-6 and IL4 of the Bispecific antibody 1 molecule with or without target cells respectively; FIG. 6F-6J show the cytokine release results of IFNy, TNF$\alpha$, IL-10, IL-6 and IL4 of the release of the Bispecific antibody 2 molecule with or without target cells respectively). The levels of cytokine release induced by Bispecific antibody 2 were weaker than that induced by Bispecific antibody 1. The levels of cytokine release induced by both bispecific antibodies were weak or absent in the presence of PBMC only, and there was a suitable safety window for co-incubation with PBMC and SHP-77 tumor cells.

### Example 5. Physical Stability Testing of Anti-CD3-DLL3 Bispecific Antibodies

NanoDSF (differential fluorescence scanning technique) was used to examine the thermal stability of different antibodies in PBS buffer at pH 7.4. Sample concentrations of approximately 1mg/ml were detected using Prometheus NT.Plex (nano DSF). Each sample was centrifuged at 10000 g for 10 minutes prior to testing. 40$\mu$l of sample was added to each well of the sample plate (the instrument loading volume was 10 $\mu$l, and each sample had one duplicate well). The scanning temperature started at 30°C and ends at 95°C, and the scanning rate was 0.5 °C/min. The experimental results are shown in Table 7. Both bispecific antibody molecules showed good thermal stability, and the initial aggregation temperature (Tagg) of the Bispecific antibody 2 molecule was higher than that of the Bispecific antibody 1 molecule.

Table 7 NanoDSF test results of anti-CD3-DLL3 bispecific antibodies

| Molecule number | Tm Onset | Tm1 | Tm2 | Tm3 | Tagg |
|---|---|---|---|---|---|
| Bispecific antibody 1 | 58.3°C | 61.5°C | 68.9°C | 81.8°C | 62.1°C |
| Bispecific antibody 2 | 58.5°C | 62.2°C | 70.1°C | / | 70.1°C |

### Example 6. Pharmacokinetics of anti-CD3-DLL3 bispecific antibodies

Two naive cynomolgus were used in the experiment and were given free access to water. The bispecific antibody was administered at a dose of 1 mg/kg, with intravenous infusion completed in 30 minutes. The blood collection time points were Pre-dose, 5 min (during infusion), 30 min (end of administration), 2hr, 4hr, 6hr, 24hr (1d), 48hr (2d), 72hr (3d), 120hr (5d), 168hr (7d), 336hr (14d), 504hr (21d), and 672hr (28d). The whole blood samples were collected in polyethylene tubes without anticoagulants, placed at room temperature for about 1 hour, centrifuged at 6000 g at 25°C, and immediately divided into two aliquots (PK sample and cytokine detection sample), immediately placed on dry ice, and transferred to a -80°C refrigerator for long-term storage.

The concentration of intact DLL3/CD3 molecules in serum was detected by ELISA. Human DLL3 protein was used to coat a 96-well plate at a concentration of 1 $\mu$g/ml, with 100 $\mu$L per well, and placed at 4°C overnight; the plate was washed 3 times with 200 $\mu$L PBST per well, 300 $\mu$L blocking reagent 5% milk powder was added, and the plate was incubated at 37°C for 1 h; the plate was washed 3 times with 200 $\mu$L PBST per well, 100 $\mu$L of the test sample was added, and the plate was incubated at 37°C for 1 h; the plate was washed 6 times with 300 $\mu$L PBST per well, 100 $\mu$L Biotin-CD3e (1: 10000), SA-HRP (1:10000) and TMB were added, and the plate was placed in the dark for 10 min, and 100 $\mu$L stop solution was added to stop the color reaction. The concentration of intact DLL3/CD3 molecules was quantitatively detected based on the color reaction.

The absorbance at 450 nm was detected by M5 plate reader from MD Company, and the data were processed using softmax software.

Phoenix Winnolin 8.2 software was used to calculate the macaques serum concentration of Bispecific antibody 2 to obtain pharmacokinetic parameters, as shown in Table 8 below.

Table 8 Macaques serum concentrations and PK parameters of Bispecific antibody 2

| Time Point (hours) | Cynomolgus 1 | Cynomolgus 2 | Average | SD | RSD (%) |
|---|---|---|---|---|---|
| 0.083 | 3.778 | 3.478 | 3.63 | 0.2 | 5.8 |
| 0.5 | 26.185 | 24.430 | 25.31 | 1.2 | 4.9 |
| 2 | 22.431 | 22.569 | 22.50 | 0.1 | 0.4 |
| 4 | 21.657 | 20.502 | 21.08 | 0.8 | 3.9 |
| 6 | 17.863 | 18.996 | 18.43 | 0.8 | 4.3 |
| 24 | 9.500 | 10.141 | 9.82 | 0.5 | 4.6 |
| 48 | 6.680 | 6.815 | 6.75 | 0.1 | 1.4 |

(continued)

| Time Point (hours) | Cynomolgus 1 | Cynomolgus 2 | Average | SD | RSD (%) |
|---|---|---|---|---|---|
| 72 | 5.054 | 5.417 | 5.24 | 0.3 | 4.9 |
| 120 | 3.323 | 3.699 | 3.51 | 0.3 | 7.6 |
| 168 | 2.479 | 2.550 | 2.51 | 0.1 | 2.0 |
| 336 | 0.891 | 0.793 | 0.84 | 0.1 | 8.2 |
| 504 | 0.327 | 0.155 | 0.24 | 0.1 | 50.5 |
| 672 | BLQ | BLQ | / | / | / |
| T1/2 (day) | 4.78 | 3.54 | 4.2 | 0.9 | 21.0 |
| T1/2 (hr) | 114.73 | 85.07 | 99.9 | 21.0 | 21.0 |
| Tmax (hr) | 0.50 | 0.50 | 0.5 | 0.0 | 0.0 |
| Cmax ($\mu$g/ml) | 26.19 | 24.43 | 25.3 | 1.2 | 4.9 |
| AUClast (day*$\mu$g/ml) | 59.72 | 61.06 | 60.4 | 0.9 | 1.6 |
| AUCINF_obs (day*$\mu$g/ml) | 61.98 | 61.85 | 61.9 | 0.1 | 0.1 |
| Vz_obs (ml/kg) | 111.28 | 82.68 | 97.0 | 20.2 | 20.9 |
| Cl_obs (ml/day/kg) | 16.13 | 16.17 | 16.2 | 0.0 | 0.1 |
| MRTINF_obs (hr) | 128.43 | 107.00 | 117.7 | 15.2 | 12.9 |

Concentrations in the above table are in $\mu$g/mL, BLQ refers to below the limit of detection.

The half-life of Bispecific antibody 2 in macaques is 99.9±21 h, Cmax is 25.3±1.2 $\mu$g/mL, AUC is 60.4±0.9 day*$\mu$g/mL, so the bispecific antibody is stable in macaques, has no obvious off-target binding, and has good pharmacokinetic properties

## Example 7. Pharmacokinetic of anti-CD3-DLL3 bispecific antibodies accompanied by cytokine detection

The detection kit is Muti-Analyte Flow assay kit (Biolegend, catalog number 740391). Before use, 250 $\mu$L of buffer was added to the freeze-dried NHP Th cytokine, mixed, and allowed to stand at room temperature for 10 minutes. The standard was diluted 1:4 with the assay buffer in the kit, and the PK serum sample was diluted 1:4 with the assay buffer in the kit. 10 mL of LEGENDplex Assay Buffer was added to lyophilized Matrix B and dissolved at room temperature for 15 minutes.

25$\mu$L Matrix B and 25$\mu$L standard were added to standard wells, and 25 $\mu$L assay buffer and 25$\mu$L serum sample were added to sample wells. The test beads were vortexed and mixed, then each test beads were mixed in 1:1 and diluted to working concentration with assay buffer, and added with 25$\mu$L to each well. The plate was sealed with sealing film and incubated at 800 rpm in the dark for 2 hours. The plate was centrifuged at 250 g for 5 minutes, the supernatant was discarded, and the plate was washed with 200 $\mu$L/well wash buffer. 25 $\mu$L of antibody to be detected was added into the plate, then the plate was sealed with sealing film, incubated at room temperature with shaking at 600 rpm in the dark for 1 hour, 25 $\mu$L of SA-PE was added, the plate was sealed with sealing film, incubated at room temperature with shaking at 600 rpm in the dark for 0.5 hour. The plate was centrifuged at 250 g for 5 minutes, the supernatant was discarded, and the plate was washed with 200 $\mu$L/well wash buffer. The samples were resuspended in 1% BSA (in PBS), filtered through 300-mesh gauze, and detected by flow cytometry.

The FCS files were exported and the cytokine release of the samples was analyzed using LENEGDplex 8.0 software. The results are shown in Table 9.

Table 9 Pharmacokinetic of anti-CD3-DLL3 bispecific antibodies accompanied by cytokine detection results

| Animal | Sampling time point | IL-2 (pg/ml) | IL-6 (pg/ml) | IL-10 (pg/ml) | TNFa (pg/ml) | IFNg (pg/ml) |
|---|---|---|---|---|---|---|
| Cynomolgus 1 | 0 h | 40.63 | <4.42 | <7.29 | <8.04 | <9.81 |
| | 2 h | 46.86 | 13.32 | 27.67 | <8.04 | <9.81 |
| | 4 h | <14.87 | <4.42 | <7.29 | <8.04 | <9.81 |
| | 6 h | <14.87 | <4.42 | <7.29 | <8.04 | <9.81 |
| | 24 h | 171.96 | 5.04 | 20.48 | <8.04 | 15.85 |
| Cynomolgus 2 | 0 h | 46.86 | <4.42 | 13.52 | <8.04 | <9.81 |
| | 2 h | 194.61 | 5.55 | 22.86 | 11.36 | 45.45 |
| | 4 h | 218.22 | 5.81 | 16.96 | 15.67 | 39.81 |
| | 6 h | 268.04 | 6.6 | 26.46 | 22.77 | 74.56 |
| | 24 h | <14.87 | <4.42 | <7.29 | <8.04 | <9.81 |

The experimental results showed that the overall release level of the pharmacokinetic accompanied by detection of various cytokines of Bispecific antibody 2 was very low, all of which were <300pg/ml, and has relatively good safety.

## Example 8. In vivo efficacy test of anti-CD3-DLL3 bispecific antibodies

All animals for experiment were kept in independent ventilation boxes with constant temperature and humidity. The temperature in the breeding room was 20.0-26.0°C, the humidity was 40-70%, and the light-dark alternation time was 12 h/12 h.

Human lung cancer cells SHP-77 were resuscitated and collected at logarithmic growth stage, culture medium was removed, the cells were washed twice with PBS and then inoculated (survival rates of SHP-77 cells before and after tumor loading were 98.8% and 96.6% respectively). The inoculation amount was $5 \times 10^6$/100 μL/mouse. After mixing with matrix gel in a 1:1 ratio, the cells were inoculated subcutaneously into NCG mice. The inoculation site was the right forelimb of the mouse (position 3 shown in FIG. 7).

PBMC were derived from normal human peripheral blood. 48 hours after inoculation of tumor cells, cryoprotectant was removed PBMC were washed twice with PBS and then transplanted into mice with $0.8 \times 10^7$/100 μL/mouse tail vein to establish a mouse model reconstructed with humanized immune cells. The cell survival rates before and after inoculation were 97.7% and 95.8%, respectively.

On the third day after tumor inoculation, when the average tumor volume reached 87.91 mm$^3$, 35 mice were randomly divided into 5 groups according to the tumor volume, including human IgG1 (AA) negative control group (purchased from Biointron, Cat. No. B109802), 0.5 mg/kg Bispecific Antibody 2 treatment group, 0.1 mg/kg Bispecific antibody 2 treatment group, 0.02 mg/kg Bispecific Antibody 2 treatment group and 0.004 mg/kg Bispecific Antibody 2 treatment group, with 7 mice in each group. The day of grouping was defined as Day D0, and administration by intraperitoneal injection was started on D0, once every three days, for a total of 6 doses (see Table 10).

Table 10 Experimental grouping and dosing regimen

| Group number | Number of mice per group | Test drug | Dose (mg/kg) | Route of administration | Actual dosing frequency cycle |
|---|---|---|---|---|---|
| G1 | 7 | hIgG1 (AA) | 0.5 | intraperitoneal administration | Once every 3 days, a total of 6 doses |
| G2 | 7 | Bispecific antibody 2 | 0.5 | intraperitoneal administration | Once every 3 days, a total of 6 doses |
| G3 | 7 | Bispecific antibody 2 | 0.1 | intraperitoneal administration | Once every 3 days, a total of 6 doses |
| G4 | 7 | Bispecific antibody 2 | 0.02 | intraperitoneal administration | Once every 3 days, a total of 6 doses |
| G5 | 7 | Bispecific antibody 2 | 0.004 | intraperitoneal administration | Once every 3 days, a total of 6 doses |

The animals' daily behavior was monitored every day after administration for 17 days. During the whole experiment, the length and width of tumor were measured twice a week with vernier calipers, and the tumor volume $(mm^3) = 0.5 \times$ (length of tumor $\times$ width of tumor $^2$) was calculated. The relative tumor growth inhibition rate TGI (%): TGI%= $(1 - T/C) \times 100\%$. T/C % was the relative tumor growth inhibition rate, i.e., the percentage ratio of tumor volume or tumor weight in the treatment group versus that in PBS control group at a certain time point. T and C were the tumor volume (TV) or tumor weight (TW) of the treatment group and PBS control group at a specific time point, respectively. The experimental results such as tumor volume, mouse body weight, tumor weight, etc. of each group of animals were expressed as mean $\pm$ standard error (Mean $\pm$ SEM). Independent sample T test was used to compare whether there were significant differences between different treatment groups and the control group. Data were analyzed using SPSS. P<0.05 indicated significant difference. The graphing software is Graphpad prism.

According to the statistical analysis of the comprehensive data of tumor volume and tumor weight at the efficacy endpoint (see Table 11, FIG. 8), compared with the hIgG 1 (AA) of control group G1, the three groups G2, G3, and G4 with higher doses (0.5 mg/kg, 0.1 mg/kg, and 0.02 mg/kg) of the test drug Bispecific antibody 2 had an inhibition rate of more than 90% on tumor volume and tumor weight, respectively, and had a very significant tumor growth inhibition effect (***P < 0.001); only group G5 with the lowest dose (0.004 mg/kg) of Bispecific antibody 2 had no significant inhibition effect on tumor growth (TGI = 17.31%, P > 0.05).

Under the current test system, compared with the hIgG1 (AA) control group G1, Bispecific antibody 2 (0.5 mg/kg) in group G2, Bispecific antibody 2 (0.1 mg/kg) in group G3, and Bispecific antibody 2 (0.02 mg/kg) in group G4 showed significant growth inhibition effects on mouse tumor volume and tumor weight; and this tumor inhibitory effect was exerted through the killing of tumors by T cells.

Table 11 Tumor volume and tumor inhibition rate in each treatment group

| Group number | Test drug | Dose (mg/kg) | Mean tumor volume (mm$^3$) | | Tumor inhibition rate% |
| --- | --- | --- | --- | --- | --- |
| | | | Day0 | Day 17 | |
| 1 | IgG1 (AA) | 0.5 | 88.00 | 1587.51 | / |
| 2 | Bispecific antibody 2 | 0.5 | 87.85 | 14.32 | 99.11 |
| 3 | Bispecific antibody 2 | 0.1 | 87.84 | 6.52 | 99.61 |
| 4 | Bispecific antibody 2 | 0.02 | 87.98 | 116.28 | 92.66 |
| 5 | Bispecific antibody 2 | 0.004 | 87.97 | 1319.62 | 17.31 |

The embodiments of the present disclosure described above are exemplary only, and any person skilled in the art will recognize or be able to identify numerous equivalents to specific compounds, materials, and procedures without preforming unconventional experimentations. All such equivalents are within the scope of the present disclosure and are encompassed by the claims.

**Claims**

1. A bispecific antigen-binding molecule comprising:

   (A) a first polypeptide comprising: (i) an antigen-binding fragment (Fab) heavy chain domain specifically directing to a first antigen, (ii) a single-chain antibody (scFv) domain capable of specifically binding to a second antigen, and (iii) a first Fc domain;
   (B) a second polypeptide comprising: a second Fc domain;
   (C) a third polypeptide comprising: an antigen-binding fragment (Fab) light chain domain specifically directing to the first antigen;

   the antigen-binding fragment (Fab) heavy chain domain and the antigen-binding fragment (Fab) light chain domain form a first binding site against the first antigen, the single-chain antibody (scFv) domain forms a second binding site against the second antigen, and the first Fc domain and the second Fc domain being associated with each other.

2. The bispecific antigen-binding molecule according to claim 1, having one or more of the following features:

   (1) the first antigen is DLL3;

preferably, the antigen-binding fragment (Fab) heavy chain domain comprises HCDR1 with the sequence as shown in SEQ ID NO: 13, HCDR2 with the sequence as shown in SEQ ID NO: 14 and HCDR3 with the sequence as shown in SEQ ID NO: 15; the antigen-binding fragment (Fab) light chain domain comprises LCDR1 with the sequence as shown in SEQ ID NO: 16, LCDR2 with the sequence as shown in SEQ ID NO: 17 and LCDR3 with the sequence as shown in SEQ ID NO: 18;

more preferably, the antigen-binding fragment (Fab) heavy chain domain comprises a first heavy chain variable region with the sequence as shown in SEQ ID NO: 11; the antigen-binding fragment (Fab) light chain domain comprises a first light chain variable region with the sequence as shown in SEQ ID NO: 12;

more preferably, the antigen-binding fragment (Fab) heavy chain domain comprises the amino acid sequence as shown in SEQ ID NO: 33; the antigen-binding fragment (Fab) light chain domain comprises the amino acid sequence as shown in SEQ ID NO: 4;

(2) the single-chain antibody (scFv) domain comprises a second heavy chain variable region and a second light chain variable region;

preferably, the second heavy chain variable region is connected to the second light chain variable region via a first linker or directly, wherein:

the C-terminal of the second heavy chain variable region is fused with the N-terminal of the first linker, and the C-terminal of the first linker is fused with the N-terminal of the second light chain variable region; or

the C-terminal of the second light chain variable region is fused with the N-terminal of the first linker and the C-terminal of the first linker is fused with the N-terminal of the second heavy chain variable region; or

the C-terminal of the second heavy chain variable region is fused with the N-terminal of the second light chain variable region; or

the C-terminal of the second light chain variable region is fused with the N-terminal of the second heavy chain variable region;

more preferably, the first linker comprises an amino acid sequence $(G_4S)_n$, wherein n is any integer from 1 to 10;

(3) the second antigen is CD3,

preferably CD3$\varepsilon$;

preferably, the single-chain antibody (scFv) domain comprises HCDR1 with the sequence as shown in SEQ ID NO: 24, HCDR2 with the sequence as shown in SEQ ID NO: 25, HCDR3 with the sequence as shown in SEQ ID NO: 26, LCDR1 with the sequence as shown in SEQ ID NO: 27, LCDR2 with the sequence as shown in SEQ ID NO: 28, and LCDR3 with the sequence as shown in SEQ ID NO: 29;

more preferably, the single-chain antibody (scFv) domain comprises a second heavy chain variable region with the sequence as shown in SEQ ID NO: 21 and a second light chain variable region with the sequence as shown in SEQ ID NO: 22;

more preferably, the single-chain antibody (scFv) domain comprises the amino acid sequence as shown in SEQ ID NO: 23;

(4) the first Fc domain comprises a first CH2 domain and a first CH3 domain of an immunoglobulin, the C-terminal of the first CH2 domain being fused with the N-terminal of the first CH3 domain; the second Fc domain comprises a second CH2 domain and a second CH3 domain of an immunoglobulin, the C-terminal of the second CH2 domain being fused with the N-terminal of the second CH3 domain;

preferably, the first CH3 domain comprises a "knob" structure and the second CH3 domain comprises a "hole" structure;

more preferably, the "knob" structure comprises amino acid substitutions S354C and T366W, the "hole" structure comprises amino acid substitutions Y349C, T366S, L368A and Y407V;

preferably, the Fc domain is derived from IgG1;

preferably, the single-chain antibody (scFv) domain is connected to the first Fc domain via a second linker or directly, wherein:

the C-terminal of the single-chain antibody (scFv) domain is fused with the N-terminal of the second linker, and the C-terminal of the second linker is fused with the N-terminal of the first Fc domain; or

the C-terminal of the single-chain antibody (scFv) domain is fused with the N-terminal of the first Fc domain;

more preferably, the second linker comprises an amino acid sequence EPKSS (SEQ ID NO: 34); more preferably, the first Fc domain comprises the amino acid sequence as shown in SEQ ID NO: 31; the second Fc domain comprises the amino acid sequence as shown in SEQ ID NO: 3;

(5) the heavy chain domain of the antigen-binding fragment (Fab) comprises a first heavy chain variable region and a CH1 domain of an immunoglobulin, and the C-terminal of the first heavy chain variable region is fused with the N-terminal of the CH1 domain; the light chain domain of the antigen-binding fragment (Fab) comprises a first light chain variable region and a light chain constant region of an immunoglobulin, and the C-terminal of the first light chain variable region is fused with the N-terminal of the light chain constant region; preferably, the antigen-binding fragment (Fab) heavy chain domain is connected to the single-chain antibody (scFv) domain by a third linker or directly, wherein:

the C-terminal of the heavy chain domain of the antigen-binding fragment (Fab) is fused with the N-terminal of the third linker, and the C-terminal of the third linker is fused with the N-terminal of the single-chain antibody (scFv) domain; or the C-terminal of the single-chain antibody (scFv) domain is fused with the N-terminal of the third linker, and the C-terminal of the third linker is fused with the N-terminal of the antigen-binding fragment (Fab) heavy chain domain; or the C-terminal of the heavy chain domain of an antigen-binding fragment (Fab) is fused with the N-terminal of the single-chain antibody (scFv) domain; or the C-terminal of the single-chain antibody (scFv) domain is fused with the N-terminal of the antigen-binding fragment (Fab) heavy chain domain; more preferably, the third linker comprises an amino acid sequence $(G_4S)_n$, wherein n is any integer from 1 to 10;

(6) the first polypeptide comprises the following structure:

Fab heavy chain domain-the third linker-scFv domain-the second linker-the first Fc domain or scFv domain-the third linker-Fab heavy chain domain-the second linker-the first Fc domain; preferably, the first polypeptide comprises the following structure:

the first heavy chain variable region-CH1-the third linker-the second heavy chain variable region-the first linker-the second light chain variable region-the second linker-the first CH2-the first CH3; or the first heavy chain variable region-CH1-the third linker-the second light chain variable region-the first linker-the second heavy chain variable region-the second linker-the first CH2-the first CH3; or the second heavy chain variable region-the first linker-the second light chain variable region-CH1-the third linker-the first heavy chain variable region-the second linker-the first CH2-the first CH3; or the second light chain variable region-the first linker-the second heavy chain variable region-CH1-the third linker-the first heavy chain variable region-the second linker-the first CH2-the first CH3; the second polypeptide comprises the following structure: the second CH2-the second CH3; the third polypeptide comprises the following structure: the first light chain variable region-light chain constant region; more preferably, the first polypeptide comprises the amino acid sequence as shown in SEQ ID NO: 2; the second polypeptide comprises the amino acid sequence as shown in SEQ ID NO: 3; the third polypeptide comprises the amino acid sequence as shown in SEQ ID NO: 4;

(7) the bispecific antigen-binding molecule comprises one or more amino acid substitutions selected from the group consisting of: (i) L234A and L235A; (ii) H435R; preferably, the H435R substitution is a substitution on the second polypeptide; and/or

(8) the epitope to which the bispecific antigen-binding molecule binds is:

(i) identical to or overlaps with an epitope to which an antibody comprising HCDR1 with the sequence as shown in SEQ ID NO: 13, HCDR2 with the sequence as shown in SEQ ID NO: 14, HCDR3 with the sequence as shown in SEQ ID NO: 15, LCDR1 with the sequence as shown in SEQ ID NO: 16, LCDR2 with the sequence as shown in SEQ ID NO: 17, and LCDR3 with the sequence as shown in SEQ ID NO: 18 directs; or (ii) identical to or overlaps with an epitope to which an antibody comprising a first heavy chain variable region with the sequence as shown in SEQ ID NO: 11 and a first light chain variable region with the sequence as shown in SEQ ID NO: 12 directs.

3. A bispecific antigen-binding molecule comprising two homologous polypeptides, each of the polypeptides comprising: (i) a nanobody (VHH) domain capable of specifically binding to a first antigen, (ii) a single-chain antibody (scFv) domain capable of specifically binding to a second antigen, and (iii) an Fc domain; the Fc domains of the two polypeptides being associated with each other.

4. The bispecific antigen-binding molecule according to claim 3, having one or more of the following features:

   (1) the first antigen is DLL3;

   > preferably, the nanobody (VHH) domain comprises HCDR1 with the sequence as shown in SEQ ID NO: 6, HCDR2 with the sequence as shown in SEQ ID NO: 7 and HCDR3 with the sequence as shown in SEQ ID NO: 8;
   > more preferably, the nanobody (VHH) domain comprises the amino acid sequence as shown in SEQ ID NO: 32;

   (2) the single-chain antibody (scFv) domain comprises a heavy chain variable region and a light chain variable region;
   preferably, the heavy chain variable region is connected to the light chain variable region via a first linker or directly, wherein:

   > the C-terminal of the heavy chain variable region is fused with the N-terminal of the first linker and the C-terminal of the first linker is fused with the N-terminal of the light chain variable region; or
   > the C-terminal of the light chain variable region is fused with the N-terminal of the first linker and the C-terminal of the first linker is fused with the N-terminal of the heavy chain variable region; or
   > the C-terminal of the heavy chain variable region is fused with the N-terminal of the light chain variable region; or
   > the C-terminal of the light chain variable region is fused with the N-terminal of the heavy chain variable region;
   > more preferably, the first linker comprises an amino acid sequence $(G_4S)_n$, wherein n is any integer from 1 to 10;

   (3) the second antigen is CD3,

   > preferably CD3$\epsilon$;
   > preferably, the single-chain antibody (scFv) domain comprises HCDR1 with the sequence as shown in SEQ ID NO: 24, HCDR2 with the sequence as shown in SEQ ID NO: 25, HCDR3 with the sequence as shown in SEQ ID NO: 26, LCDR1 with the sequence as shown in SEQ ID NO: 27, LCDR2 with the sequence as shown in SEQ ID NO: 28, and LCDR3 with the sequence as shown in SEQ ID NO: 29;
   > more preferably, the single-chain antibody (scFv) domain comprises a heavy chain variable region with the sequence as shown in SEQ ID NO: 21 and a light chain variable region with the sequence as shown in SEQ ID NO: 22;
   > more preferably, the single-chain antibody (scFv) domain comprises an amino acid sequence shown in SEQ ID NO: 23;

   (4) the Fc domain comprises a CH2 domain and a CH3 domain of an immunoglobulin, the C-terminal of the CH2 domain being fused with the N-terminal of the CH3 domain;

   > preferably, the Fc domain is derived from IgG1;
   > preferably, the Fc domain is connected to the single-chain antibody (scFv) domain via a second linker or directly, wherein:

   > > the C-terminal of the single-chain antibody (scFv) domain is fused with the N-terminal of the second linker, and the C-terminal of the second linker is fused with the N-terminal of the Fc domain; or
   > > the C-terminal of the single-chain antibody (scFv) domain is fused with the N-terminal of the Fc domain;
   > > more preferably, the second linker comprises an amino acid sequence EPKSS (SEQ ID NO: 34);
   > > more preferably, the Fc domain comprises the amino acid sequence as shown in SEQ ID NO: 30;

   (5) the nanobody (VHH) domain is connected to the single-chain antibody (scFv) domain by a third linker or directly, wherein:

the C-terminal of the nanobody (VHH) domain is fused with the N-terminal of the third linker, and the C-terminal of the third linker is fused with the N-terminal of the single-chain antibody (scFv) domain; or

the C-terminal of the single-chain antibody (scFv) domain is fused with the N-terminal of the third linker, and the C-terminal of the third linker is fused with the N-terminal of nanobody (VHH) domain; or

the C-terminal of the nanobody (VHH) domain is fused with the N-terminal of the single-chain antibody (scFv) domain; or

the C-terminal of the single-chain antibody (scFv) domain is fused with the N-terminal of nanobody (VHH) domain;

more preferably, the third linker comprises an amino acid sequence $(G_4S)_n$, wherein n is any integer from 1 to 10;

(6) each of the polypeptides comprises the following structure:

VHH domain-the third linker-scFv domain-the second linker-Fc domain or scFv domain-the third linker-VHH domain-the second linker-Fc domain;

preferably, each of the polypeptides comprises the following structure:

VHH domain-the third linker-heavy chain variable region-the first linker-light chain variable region-the second linker-CH2-CH3, or

VHH domain-the third linker-light chain variable region-the first linker-heavy chain variable region-the second linker-CH2-CH3, or

heavy chain variable region-the first linker-light chain variable region-the third linker-VHH domain-the second linker-CH2-CH3, or

light chain variable region-the first linker-heavy chain variable region-the third linker-VHH domain-the second linker-CH2-CH3;

more preferably, each of the polypeptide comprises the amino acid sequence as shown in SEQ ID NO: 1;

(7) the bispecific antigen-binding molecule comprises the following amino acid substitutions: L234A and L235A; and/or

(8) the epitope to which the bispecific antigen-binding molecule binds is:

(i) identical to or overlaps with an epitope to which a nanobody comprising HCDR1 with the sequence as shown in SEQ ID NO: 6, HCDR2 with the sequence as shown in SEQ ID NO: 7, HCDR3 with the sequence as shown in SEQ ID NO: 8 directs; or

(ii) identical to or overlaps with an epitope to which a nanobody comprising the amino acid sequence as shown in SEQ ID NO: 32 directs.

5. A bispecific antigen-binding molecule comprising:

(A) a first binding portion capable of specifically binding to DLL3; and
(B) a second binding portion, the antigen or epitope to which the second binding portion specifically binds is different from that of the first binding portion;

the first binding portion comprises:

(i) HCDR1 with the sequence as shown in SEQ ID NO: 6, HCDR2 with the sequence as shown in SEQ ID NO: 7, and HCDR3 with the sequence as shown in SEQ ID NO: 8; or

(ii) HCDR1 with the sequence as shown in SEQ ID NO: 13, HCDR2 with the sequence as shown in SEQ ID NO: 14, HCDR3 with the sequence as shown in SEQ ID NO: 15, LCDR1 with the sequence as shown in SEQ ID NO: 16, LCDR2 with the sequence as shown in SEQ ID NO: 17, and LCDR3 with the sequence as shown in SEQ ID NO: 18; or

(iii) the amino acid sequence as shown in SEQ ID NO: 32; or

(iv) a first heavy chain variable region with the sequence as shown in SEQ ID NO: 11 and a first light chain variable region with the sequence as shown in SEQ ID NO: 12.

6. A nucleic acid encoding the bispecific antigen-binding molecule according to any one of the preceding claims, or an expression vector comprising the nucleic acid, or a host cell comprising the nucleic acid or the expression vector; preferably, the host cell is a prokaryotic cell (preferably Escherichia coli), or a eukaryotic cell (preferably a mammalian cell or yeast; more preferably, the mammalian cell is a CHO cell, Expi293 or HEK293 cell).

7. A method for preparing the bispecific antigen-binding molecule according to any one of claims 1 to 5, comprising culturing the host cell according to claim 6 under a suitable condition.

8. An antibody-drug conjugate forming by conjugating the bispecific antigen-binding molecule according to any one of claims 1 to 5 with other biologically active molecule; preferably, the other biologically active molecule is a small molecule drug; preferably, the bispecific antigen-binding molecule and the other biologically active molecule are connected via a linker.

9. A pharmaceutical composition comprising the bispecific antigen binding molecule according to any one of claims 1 to 5, or the nucleic acid, expression vector or host cell according to claim 6, or the antibody-drug conjugate according to claim 8;

   preferably, the pharmaceutical composition further comprises a pharmaceutically acceptable carrier;
   preferably, the pharmaceutical composition further comprises one or more additional therapeutic agents.

10. Use of the bispecific antigen binding molecule according to any one of claims 1 to 5, the nucleic acid, expression vector or host cell according to claim 6, or the antibody-drug conjugate according to claim 8 in the manufacture of a medicament for treating, alleviating and/or preventing a tumor;
    preferably, the tumor is a DLL3-positive tumor; more preferably, the tumor is selected from: small cell lung cancer, glioblastoma, neuroendocrine cancer, melanoma, pancreatic cancer, rectal cancer and a metastatic cancer of the above-mentioned tumor.

11. A method for inducing death of a cell expressing DLL3, the method comprises contacting the cell with the bispecific antigen binding molecule according to any one of claims 1 to 5, the nucleic acid, expression vector or host cell according to claim 6, the antibody-drug conjugate according to claim 8, or the pharmaceutical composition according to claim 9, wherein the cell expressing DLL3 is a tumor cell;
    preferably, the tumor cell is a cell selected from the following tumors: small cell lung cancer, glioblastoma, neuroendocrine cancer, melanoma, pancreatic cancer, rectal cancer and a metastatic cancer of the above-mentioned tumor.

12. A method for treating a disease associated with expression of DLL3 in a subject, the method comprises administering the bispecific antigen binding molecule according to any one of claims 1 to 5, the nucleic acid, expression vector or host cell according to claim 6, the antibody-drug conjugate according to claim 8, or the pharmaceutical composition according to claim 9 to a subject in need thereof;

    preferably, the disease is a tumor; more preferably, the tumor is small cell lung cancer, glioblastoma, neuroendocrine cancer, melanoma, pancreatic cancer, rectal cancer and a metastatic cancer of the above-mentioned tumor;
    preferably, the method further comprises administering an additional therapeutic agent to the subject.

Bispecific antibody 1

Bispecific antibody 2

◓  Anti-DLL3 nanobody

◓  Light chain variable region of anti-CD3 antibody

◓  Heavy chain variable region of anti-CD3 antibody

◓  Fc domain

◓  Fab fragment of anti-DLL3 full-length antibody

◓  Light chain variable region of anti-CD3 antibody

◓  Heavy chain variable region of anti-CD3 antibody

◓  Fc domain with "knob" and "hole" structures

FIG. 1A

FIG. 1B

FIG. 1

FIG. 2

FIG. 3

FIG. 4

FIG. 5

FIG. 6A

FIG. 6B

FIG. 6C

FIG. 6D

FIG. 6E

FIG. 6F

FIG. 6G

FIG. 6H

FIG. 6I

FIG. 6J

FIG. 6

FIG. 7

FIG. 8

**INTERNATIONAL SEARCH REPORT**

International application No.

**PCT/CN2023/075148**

### A. CLASSIFICATION OF SUBJECT MATTER

C07K 16/46(2006.01)i;C12N 15/13(2006.01)i;C12N 15/63(2006.01)i;A61K 39/395(2006.01)i;A61P 35/00(2006.01)i

According to International Patent Classification (IPC) or to both national classification and IPC

### B. FIELDS SEARCHED

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

CNKI, CNABS, CNTXT, WPABSI, WPABSC, ENTXTC, ENTXT, ISI web of science and Search terms: CD3, DLL3, 抗体, 双特异, antibod+, bispecific, VHH, scFv, Fab, Fc etc.; GENBANK, EMBL, 中国专利生物序列检索系统和检索的序列: SEQ ID NOs: 1-34, China Patent Biological Sequence Search System and searched sequences: SEQ ID NOs: 1-34.

### C. DOCUMENTS CONSIDERED TO BE RELEVANT

| Category* | Citation of document, with indication, where appropriate, of the relevant passages | Relevant to claim No. |
|---|---|---|
| X | US 2020087412 A1 (SHANGHAI TONGJI HOSPITAL) 19 March 2020 (2020-03-19) claims 1-22, description paragraphs [0076]-[0080] | 1, 2, 6-10 |
| A | US 2020087412 A1 (SHANGHAI TONGJI HOSPITAL) 19 March 2020 (2020-03-19) claims 1-22, description paragraphs [0076]-[0080] | 3-5 |
| Y | WO 2021115456 A1 (WUXI BIOLOGICS SHANGHAI CO., LTD. et al.) 17 June 2021 (2021-06-17) claims 1-19 | 3, 4, 6-10 |
| A | WO 2021115456 A1 (WUXI BIOLOGICS SHANGHAI CO., LTD. et al.) 17 June 2021 (2021-06-17) claims 1-19 | 1, 2, 5 |
| Y | CN 108271376 A (AMGEN RESEARCH (MUNICH) GMBH) 10 July 2018 (2018-07-10) claims 1-28 | 3, 4, 6-10 |
| A | CN 108271376 A (AMGEN RESEARCH (MUNICH) GMBH) 10 July 2018 (2018-07-10) claims 1-28 | 1, 2, 5 |

☑ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 May 2023** | **27 May 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/075148** |

**C.     DOCUMENTS CONSIDERED TO BE RELEVANT**

| Category* | Citation of document, with indication, where appropriate, of the relevant passages | Relevant to claim No. |
|---|---|---|
| A | CN 112513092 A (BOEHRINGER INGELHEIM INTERNATIONAL GMBH) 16 March 2021 (2021-03-16)<br>claims 1-47 | 1-10 |
| A | WO 2021155380 A1 (GENSUN BIOPHARMA INC.) 05 August 2021 (2021-08-05)<br>claims 1-97 | 1-10 |
| PX | WO 2022237647 A1 (SHANGHAI QILU PHARMACEUTICAL RES AND DEVELOPMENT CENTRE LTD.) 17 November 2022 (2022-11-17)<br>claims 1-14 | 5-10 |

Form PCT/ISA/210 (second sheet) (July 2022)

**INTERNATIONAL SEARCH REPORT**

International application No.

**PCT/CN2023/075148**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| **INTERNATIONAL SEARCH REPORT** | International application No. |
| | **PCT/CN2023/075148** |

**Box No. II          Observations where certain claims were found unsearchable (Continuation of item 2 of first sheet)**

This international search report has not been established in respect of certain claims under Article 17(2)(a) for the following reasons:

1. ☑ Claims Nos.: **11-12**
   because they relate to subject matter not required to be searched by this Authority, namely:

   PCT Rule 39.1(iv) – methods for treatment of the human or animal body by surgery or therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III          Observations where unity of invention is lacking (Continuation of item 3 of first sheet)**

This International Searching Authority found multiple inventions in this international application, as follows:

Invention 1: claims 1-2 (all) and 6-10 (in part) relate to a bispecific antigen-binding molecule including a first polypeptide, a second polypeptide and a third polypeptide, and an encoding nucleic acid, a preparation method, a drug conjugate, a pharmaceutical composition and the use thereof.

Invention 2: claims 3-4 (all) and 6-10 (in part) relate to a bispecific antigen-binding molecule including two homologous polypeptides, and an encoding nucleic acid, a preparation method, a drug conjugate, a pharmaceutical composition and the use thereof.

Invention 3: claims 5 (all) and 6-10 (in part) relate to a bispecific antigen-binding molecule including a first binding moiety and a second binding moiety which are capable of specifically binding to DLL3, and an encoding nucleic acid, a preparation method, a drug conjugate, a pharmaceutical composition and the use thereof.

The same or corresponding technical feature of the three inventions is a bispecific antigen-binding molecule that specifically binds to DLL3. However, the prior art (for example, CN 108271376 A) discloses a bispecific antigen-binding molecule that specifically binds to DLL3 and CD3, that is, the above-mentioned same or corresponding technical feature is disclosed in the prior art, and is not the special technical feature that makes a contribution over the prior art. Therefore, inventions 1-3 do not fall within a single general inventive concept, and thus lack unity of invention and do not comply with PCT Rule 13.1.

Form PCT/ISA/210 (continuation of first sheet) (July 2022)

|  |  |
|---|---|
| **INTERNATIONAL SEARCH REPORT** | International application No. |
|  | **PCT/CN2023/075148** |

| **Box No. III    Observations where unity of invention is lacking (Continuation of item 3 of first sheet)** |
|---|

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

**INTERNATIONAL SEARCH REPORT**
**Information on patent family members**

International application No.

**PCT/CN2023/075148**

| Patent document cited in search report | | | Publication date (day/month/year) | Patent family member(s) | | | Publication date (day/month/year) |
|---|---|---|---|---|---|---|---|
| US | 2020087412 | A1 | 19 March 2020 | US | 11066476 | B2 | 20 July 2021 |
| WO | 2021115456 | A1 | 17 June 2021 | US | 2023035169 | A1 | 02 February 2023 |
| | | | | JP | 2023505724 | A | 10 February 2023 |
| | | | | AU | 2020402496 | A1 | 16 June 2022 |
| | | | | BR | 112022010824 | A2 | 23 August 2022 |
| | | | | TW | 202136292 | A | 01 October 2021 |
| | | | | EP | 4073124 | A1 | 19 October 2022 |
| | | | | CA | 3158047 | A1 | 17 June 2021 |
| | | | | KR | 20220113382 | A | 12 August 2022 |
| CN | 108271376 | A | 10 July 2018 | IL | 296409 | A | 01 November 2022 |
| | | | | EP | 3328889 | A1 | 06 June 2018 |
| | | | | EP | 3328889 | B1 | 23 June 2021 |
| | | | | CL | 2020003046 | A1 | 09 April 2021 |
| | | | | US | 2017037130 | A1 | 09 February 2017 |
| | | | | US | 10294300 | B2 | 21 May 2019 |
| | | | | ES | 2884211 | T3 | 10 December 2021 |
| | | | | JP | 2021000088 | A | 07 January 2021 |
| | | | | JP | 6827583 | B2 | 10 February 2021 |
| | | | | CL | 2018000267 | A1 | 05 October 2018 |
| | | | | ZA | 202006983 | B | 29 March 2023 |
| | | | | US | 2020332002 | A1 | 22 October 2020 |
| | | | | US | 11591396 | B2 | 28 February 2023 |
| | | | | HUE | 055112 | T2 | 28 October 2021 |
| | | | | AU | 2016302569 | A1 | 07 December 2017 |
| | | | | AU | 2016302569 | B2 | 25 August 2022 |
| | | | | BR | 112018000475 | A2 | 18 September 2018 |
| | | | | JP | 2021061859 | A | 22 April 2021 |
| | | | | JP | 7166368 | B2 | 07 November 2022 |
| | | | | MA | 42530 | A | 24 March 2021 |
| | | | | MA | 42530 | B1 | 30 September 2021 |
| | | | | LT | 3328889 | T | 10 August 2021 |
| | | | | TW | 201708261 | A | 01 March 2017 |
| | | | | KR | 20180033501 | A | 03 April 2018 |
| | | | | KR | 102404077 | B1 | 02 June 2022 |
| | | | | US | 2021309743 | A1 | 07 October 2021 |
| | | | | TN | 2017000549 | A1 | 12 April 2019 |
| | | | | NZ | 737339 | A | 30 September 2022 |
| | | | | SG | 10201911548 | QA | 30 January 2020 |
| | | | | EP | 3865514 | A1 | 18 August 2021 |
| | | | | MY | 184895 | A | 30 April 2021 |
| | | | | CR | 20180066 | A | 29 May 2018 |
| | | | | WO | 2017021349 | A1 | 09 February 2017 |
| | | | | SI | 3328889 | T1 | 30 September 2021 |
| | | | | KR | 20220075455 | A | 08 June 2022 |
| | | | | HK | 1249525 | A1 | 02 November 2018 |
| | | | | RS | 62248 | B1 | 30 September 2021 |
| | | | | JP | 2018527908 | A | 27 September 2018 |
| | | | | JP | 6824245 | B2 | 03 February 2021 |
| | | | | US | 2019263907 | A1 | 29 August 2019 |
| | | | | US | 10683351 | B2 | 16 June 2020 |

Form PCT/ISA/210 (patent family annex) (July 2022)

INTERNATIONAL SEARCH REPORT
Information on patent family members

International application No.

**PCT/CN2023/075148**

| Patent document cited in search report | | | Publication date (day/month/year) | Patent family member(s) | | | Publication date (day/month/year) |
|---|---|---|---|---|---|---|---|
| | | | | CO | 2018000877 | A2 | 19 April 2018 |
| | | | | AU | 2022268314 | A1 | 15 December 2022 |
| | | | | PT | 3328889 | T | 25 August 2021 |
| | | | | MX | 2018001154 | A | 23 May 2018 |
| | | | | HRP | 20211073 | T1 | 01 October 2021 |
| | | | | JP | 2022191477 | A | 27 December 2022 |
| | | | | MD | 3328889 | T2 | 31 October 2021 |
| | | | | PH | 12018500241 | A1 | 13 August 2018 |
| | | | | IL | 256871 | A | 29 March 2018 |
| | | | | IL | 256871 | B | 01 October 2022 |
| | | | | CA | 2986848 | A1 | 09 February 2017 |
| | | | | DK | 3328889 | T3 | 23 August 2021 |
| | | | | PL | 3328889 | T3 | 06 December 2021 |
| | | | | PE | 20181152 | A1 | 17 July 2018 |
| | | | | JO | 3671 | B1 | 27 August 2020 |
| CN | 112513092 | A | 16 March 2021 | IL | 279235 | A | 31 January 2021 |
| | | | | KR | 20210018311 | A | 17 February 2021 |
| | | | | MX | 2020013348 | A | 09 March 2021 |
| | | | | CA | 3099956 | A1 | 12 December 2019 |
| | | | | ECSP | 20084864 | A | 31 May 2021 |
| | | | | JOP | 13 March 2020 | A1 | 06 December 2020 |
| | | | | PH | 12020552080 | A1 | 31 May 2021 |
| | | | | CL | 2020003105 | A1 | 11 June 2021 |
| | | | | CR | 20200601 | A | 26 January 2021 |
| | | | | MA | 52742 | A | 14 April 2021 |
| | | | | BR | 112020022898 | A2 | 23 February 2021 |
| | | | | JP | 2021526816 | A | 11 October 2021 |
| | | | | JP | 7133043 | B2 | 07 September 2022 |
| | | | | US | 2022251236 | A1 | 11 August 2022 |
| | | | | AU | 2019280900 | A1 | 19 November 2020 |
| | | | | US | 2019375849 | A1 | 12 December 2019 |
| | | | | US | 11332541 | B2 | 17 May 2022 |
| | | | | CO | 2020015172 | A2 | 21 December 2020 |
| | | | | EP | 3802598 | A1 | 14 April 2021 |
| | | | | PE | 20210321 | A1 | 16 February 2021 |
| | | | | JP | 2022180379 | A | 06 December 2022 |
| | | | | SG | 11202011134 | XA | 30 December 2020 |
| | | | | TW | 202016151 | A | 01 May 2020 |
| | | | | WO | 2019234220 | A1 | 12 December 2019 |
| WO | 2021155380 | A1 | 05 August 2021 | AU | 2021213840 | A1 | 29 September 2022 |
| | | | | US | 2021246222 | A1 | 12 August 2021 |
| | | | | JP | 2023512297 | A | 24 March 2023 |
| | | | | KR | 20220162690 | A | 08 December 2022 |
| | | | | EP | 4097135 | A1 | 07 December 2022 |
| WO | 2022237647 | A1 | 17 November 2022 | | None | | |

Form PCT/ISA/210 (patent family annex) (July 2022)

**EP 4 477 670 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022101238168
- US 5731168 A
- US 7695936 B